# EUROPEAN PATENT APPLICATION

(11) **EP 2 923 643 A1**
(43) Date of publication of application: **30.09.2015**
(21) Application number: 13856563.5
(22) Date of filing: 07.11.2013
(51) Int. Cl.: A61B 5/16, A61M 21/00

(54) **SYSTEM FOR SUPPORTING CORRECTION OF DISTORTED COGNITION, METHOD OF ELICITING USER CONSCIOUSNESS INFORMATION AND PROGRAM THEREFOR**

(30) Priority: 21.11.2012 JP 2012255033
(71) Applicant: NEC Solution Innovators, Ltd., Tokyo 136-8627 (JP)
(72) Inventor: ONO, Yutaka, Tokyo 157-0066 (JP); YAMAGUCHI, Mineko, Tokyo 1368627 (JP); YAMAMOTO, Kosuke, Tokyo 1368627 (JP); SHINKAI, Atsushi, Tokyo 1368627 (JP); TAGUCHI, Takehiro, Tokyo 1368627 (JP); NISHII, Kazutomo, Tokyo 1368627 (JP); TAKECHI, Sayuri, Tokyo 1368627 (JP)
(74) Representative: Betten & Resch
(86) International application number: PCT/JP2013/006566
(87) International publication number: WO 2014/080585

(57) **Abstract**

This system involves carrying out at least an automatic thought extraction step which involves processing for outputting a message which, in a situation in which the user's feelings are perturbed, prompts the input of automatic thoughts, which are thoughts occurring to the user, and an adaptive thought extraction step which includes processing for outputting a message for prompting input of adaptive thoughts, which are thoughts that could be thought by said user in regard to said situation, wherein the system is provided with message output means 101 which outputs dialogue messages, which are messages for eliciting the necessary information from the user in each step, and message generation means 102 which uses the information previously inputted by the user to generate dialogue messages in at least one of the steps.

## Description

### [Technical Field]

The present invention relates to a system for supporting correction of a cognitive distortion, a method for acquiring user consciousness information, and a program for supporting correction of a cognitive distortion, providing, to a user, support for correcting the cognitive distortion of the user or for allowing the user to be aware the cognitive distortion based on cognitive behavior therapy.

### [Background Art]

Cognitive reframing is known as an example of cognitive behavior therapy, which is a methodology for correcting the habit of the way of thinking which concentrates toward negative thinking, to the way of balanced thinking, by focusing on a fact and a behavior. In the cognitive reframing, a produced feeling, a thought, the reason of the thought, and the like in a situation in which a client is perturbed are successively written by client. Thereby, the client can reconsider the way of thinking.

Mental health care based on the cognitive reframing has been systematized and provided as service to a user (for example, NPL 1).

In addition, PTL 1 describes a mental health care system that acquires and measures the stress condition of a user in an interactive mode. In such a mental health care system, the measurement results are used to conduct counseling to the user in an interactive mode.

### [Citation List]

### [Patent Literature]

[PTL 1] Japanese Patent Application Laid-Open No. 2005-334205

### [Non Patent Literature]

[NPL 1] "Depression/Anxiety Web" [online] <URL: http://www.cbtjp.net/> on the Internet, operated by Woman Wave Co., Ltd. and supervised by Yutaka Ono, [searched on August 23, 2012]

### [Summary of Invention]

### [Technical Problem]

There have been problems that usability, functions, and the like are insufficient when counseling based on cognitive behavior therapy is fully automated and systematized. In particular, there has been a problem that it is difficult for a user to write alone the user's feeling or thought that necessary for counseling, or it is impossible for a user to objectively write those feeling or thought.

The cognitive behavior therapy is characterized by dealing with the consciousness of a client, unlike psychoanalysis which deals with unconsciousness. Therefore, the point of the therapy is how to elicit information or a solution necessary to solve a matter from a client.

It is important, for eliciting information or a solution necessary to solve a matter from a client, to establish such a therapeutic relationship between a client and a system, that the expectations of "being able to obtain something if talking" and "being able to resolve worry" are given by user. In addition, it is important that the system is able to response appropriately for allowing the client to compensate for an uncertain point of an input from the client.

The mental health care systems described in PTL 1 and NPL 1 are implemented in a form that those systems prepare predetermined questions and successively show prepared questions to be answered. Therefore, in these technologies, it can not necessarily be considered that those systems are able to elicit information that may lead solutions from clients. For improving counseling effects, it is necessary to support description of users, and to contrive user interfaces.

Thus, an objective of the present invention is to provide a system for supporting correction of a cognitive distortion, a method for acquiring user consciousness information, and a program for a system for supporting correction of a cognitive distortion, in which necessary information can be sufficiently elicited from a client even when fully automated support for correcting the cognitive distortion of a user or for allowing the user to find the cognitive distortion based on cognitive behavior therapy is performed.

### [Solution to Problem]

A system, for supporting correction of a cognitive distortion, according to the present invention is an interactive system that executes an automatic thought eliciting step including processing for outputting a message for prompting input of an automatic thought which is a thought generated in a user in a situation in which a user's feeling is perturbed and an adaptive thought eliciting step including processing for outputting a message for prompting input of an adaptive thought which is a thought that can be currently taken by the user with regard to the situation, wherein the system includes: message output means that outputs an interactive message which is a message for eliciting necessary information from the user in each of the steps; and message generation means that generates the interactive message in at least one step of the steps by utilizing information previously input by the user.

In addition, a system for supporting correction of a cognitive distortion according to the present invention is an interactive system for supporting correction of a cognitive distortion, executing an automatic thought eliciting step including processing for outputting a message for prompting input of an automatic thought which is a thought generated in a user in a situation in which a user's feeling is perturbed and an adaptive thought eliciting step including processing for outputting a message for prompting input of an adaptive thought which is a thought that can be currently taken by the user with regard to the situation, wherein the system includes flow controlling means that controls a sequence of execution of each step, wherein the flow controlling means carries out at least the automatic thought eliciting step and the adaptive thought eliciting step in this sequence as a basic flow, and may also carry out flow control of, discontinuing the automatic thought eliciting step or the adaptive thought eliciting step, when determining that user input does not proceed in the automatic thought eliciting step or the adaptive thought eliciting step, and proceeding to a predetermined step including processing for outputting a message for prompting input of information for an opportunity or hint for input by a user in the discontinued step.

In addition, a system for supporting correction of a cognitive distortion according to the present invention includes a screen for eliciting, from a user, information about a situation in which a user's feeling is perturbed, wherein the screen includes an entry field for writing a scene and an entry field for writing an event, which may be separately disposed.

In addition, a system for supporting correction of a cognitive distortion according to the present invention includes a screen for eliciting, from a user, information about a situation that a user's feeling is perturbed, wherein the screen includes an entry field in which the user can make an entry and an apparent space available for the entry may be limited to a size with not more than the predetermined number of characters.

In addition, a system for supporting correction of a cognitive distortion according to the present invention includes a screen for eliciting, from a user, an automatic thought which is a thought generated in the user in a situation in which a user's feeling is perturbed, wherein the screen may include a screen including a plurality of entry fields for allowing a user to enter an automatic thought and a screen including a selection function for, when a plurality of automatic thoughts are entered, selecting one of the automatic thoughts.

In addition, a system for supporting correction of a cognitive distortion according to the present invention includes a screen for eliciting, from a user, an adaptive thought which is a thought that can be currently taken by the user with regard to a situation in which a user's feeling is perturbed, wherein an example sentence of the adaptive thought, which is generated from a ground which is a reason for generating an automatic thought which has been previously acquired from the user and is a thought generated in the user in the situation in which the user's feeling is perturbed, or the ground and counterevidence against the automatic thought, may be displayed on the screen.

In addition, a system for supporting correction of a cognitive distortion according to the present invention includes a screen for eliciting, from a user, a ground of an automatic thought which is a thought generated in the user in a situation in which a user's feeling is perturbed, wherein a sentence in which a copulative conjunction and an summarization expression of the automatic thought are joined may be displayed in form of following an entry field for a ground on the screen.

In addition, a method for eliciting user consciousness information according to the present invention is a method for eliciting user consciousness information, applied to an interactive system for supporting correction of a cognitive distortion, executing an automatic thought eliciting step including processing for outputting a message for prompting input of an automatic thought which is a thought generated in a user in a situation in which a user's feeling is perturbed and an adaptive thought eliciting step including processing for outputting a message for prompting input of an adaptive thought which is a thought that can be currently taken by the user with regard to the situation, the method for eliciting user consciousness information, including: generating an interactive message which is a message for eliciting necessary information from the user by utilizing information previously input by the user in at least one step of the steps; and outputting the generated interactive message.

In addition, a method for eliciting user consciousness information according to the present invention is a method for eliciting user consciousness information, applied to an interactive system for supporting correction of a cognitive distortion, executing an automatic thought eliciting step including processing for outputting a message for prompting input of an automatic thought which is a thought generated in a user in a situation in which a user's feeling is perturbed and an adaptive thought eliciting step including processing for outputting a message for prompting input of an adaptive thought which is a thought that can be currently taken by the user with regard to the situation, wherein flow controlling means that controls a sequence of execution of each step carries out the automatic thought eliciting step and the adaptive thought eliciting step in this sequence in a basic flow and may also carry out flow control in which when determining that user input does not proceed in the automatic thought eliciting step or the adaptive thought eliciting step, the step is discontinued to shift to a predetermined step including processing for outputting a message for prompting input of information for an opportunity or hint for input by a user in the discontinued step.

In addition, a program for supporting correction of a cognitive distortion according to the present invention is a program for supporting correction of a cognitive distortion, applied to an interactive system for supporting correction of a cognitive distortion, executing an automatic thought eliciting step including processing for outputting a message for prompting input of an automatic thought which is a thought generated in a user in a situation in which a user's feeling is perturbed and an adaptive thought eliciting step including processing for outputting a message for prompting input of an adaptive thought which is a thought that can be currently taken by the user with regard to the situation, wherein a computer is allowed to execute: message output processing for outputting an interactive message which is a message for eliciting necessary information from the user in each of the steps; and message generation processing for generating the interactive message in at least one step of the steps by utilizing information previously input by the user.

In addition, a program for supporting correction of a cognitive distortion according to the present invention is a program for supporting correction of a cognitive distortion, applied to an interactive system for supporting correction of a cognitive distortion, executing an automatic thought eliciting step including processing for outputting a message for prompting input of an automatic thought which is a thought generated in a user in a situation in which a user's feeling is perturbed and an adaptive thought eliciting step including processing for outputting a message for prompting input of an adaptive thought which is a thought that can be currently taken by the user with regard to the situation, wherein a computer may be allowed to execute the automatic thought eliciting step and the adaptive thought eliciting step in this sequence in a basic flow and to execute flow control processing in which when determining that user input does not proceed in the automatic thought eliciting step or the adaptive thought eliciting step, the step is discontinued to shift to a predetermined step including processing for outputting a message for prompting input of information for an opportunity or hint for input by a user in the discontinued step.

### [Advantageous Effects of Invention]

In accordance with the present invention, necessary information can be sufficiently elicited from a client.

### [Brief Description of Drawings]

[Fig. 1] Fig. 1 is a block diagram illustrating a configuration example of a mental health care system of the first exemplary embodiment of the present invention.
[Fig. 2] Fig. 2 is an explanatory drawing illustrating an example of a screen used in a situation eliciting step.
[Fig. 3] Fig. 3 is an explanatory drawing illustrating an example of a screen used in a situation eliciting step.
[Fig. 4] Fig. 4 is an explanatory drawing illustrating an example of the flow of self/others determination processing in Japanese.
[Fig. 5] Fig. 5 is an explanatory drawing illustrating examples of integrated determination results and examples of input sentences in a situation eliciting step.
[Fig. 6] Fig. 6 is an explanatory drawing illustrating an example of a screen used in an automatic thought eliciting step.
[Fig. 7] Fig. 7 is an explanatory drawing illustrating an example of a screen used in an automatic thought eliciting step.
[Fig. 8] Fig. 8 is an explanatory drawing illustrating an example of a dictionary used in estimation of a feeling.
[Fig. 9] Fig. 9 is an explanatory drawing illustrating an example of a screen used in an adaptive thought eliciting step.
[Fig. 10] Fig. 10 is an explanatory drawing illustrating an example of a screen used in an adaptive thought eliciting step.
[Fig. 11] Fig. 11 is an explanatory drawing illustrating examples of hint sentences according to category classification of automatic thoughts.
[Fig. 12] Fig. 12 is an explanatory drawing illustrating an example of a dictionary in which an expression example of a preconceived expression according to each classification item is registered.
[Fig. 13] Fig. 13 is an explanatory drawing illustrating an example of the hint sentence according to each classification item of a preconceived expression.
[Fig. 14] Fig. 14 is an explanatory drawing illustrating an example of a screen used in a mood eliciting step.
[Fig. 15] Fig. 15 is an explanatory drawing illustrating an example of a screen used in a mood eliciting step.
[Fig. 16] Fig. 16 is an explanatory drawing illustrating an example of a screen used in a ground eliciting step in a ground/counterevidence eliciting step.
[Fig. 17] Fig. 17 is an explanatory drawing illustrating an example of a screen used in a ground eliciting step in a ground/counterevidence eliciting step.
[Fig. 18] Fig. 18 is an explanatory drawing illustrating an example of a screen used in a counterevidence eliciting step in a ground/counterevidence eliciting step.
[Fig. 19] Fig. 19 is an explanatory drawing illustrating an example of a screen used in a counterevidence eliciting step in a ground/counterevidence eliciting step.
[Fig. 20] Fig. 20 is a flowchart illustrating an example of operation of the mental health care system of the first exemplary embodiment.
[Fig. 21] Fig. 21 is an explanatory drawing collectively illustrating representatives of contrivance of UI and contrivance of interactive functions according to each step.
[Fig. 22] Fig. 22 is an explanatory drawing illustrating examples of an indicator.
[Fig. 23] Fig. 23 is a block diagram illustrating the outline of the present invention.
[Fig. 24] Fig. 24 is a block diagram illustrating another configuration example of a system for supporting correction of a cognitive distortion according to the present invention.
[Fig. 25] Fig. 25 is a block diagram illustrating another configuration example of a system for supporting correction of a cognitive distortion according to the present invention.
[Fig. 26] Fig. 26 is a block diagram illustrating another configuration example of a system for supporting correction of a cognitive distortion according to the present invention.
[Fig. 27] Fig. 27 is a block diagram illustrating another configuration example of a system for supporting correction of a cognitive distortion according to the present invention.
[Fig. 28] Fig. 28 is a block diagram illustrating another configuration example of a system for supporting correction of a cognitive distortion according to the present invention.
[Fig. 29] Fig. 29 is a block diagram illustrating another configuration example of a system for supporting correction of a cognitive distortion according to the present invention.
[Fig. 30] Fig. 30 is a block diagram illustrating another configuration example of a system for supporting correction of a cognitive distortion according to the present invention.
[Fig. 31] Fig. 31 is a block diagram illustrating another configuration example of a system for supporting correction of a cognitive distortion according to the present invention.
[Fig. 32] Fig. 32 is a block diagram illustrating another configuration example of a system for supporting correction of a cognitive distortion according to the present invention.

### [Description of Embodiments]

First, the definitions of terms will be explained. In cognitive reframing, a thought generated in a client in a situation in which the client is perturbed is referred to as "automatic thought". When a cognitive distortion is generated in the automatic thought, the client may mistakenly recognize a fact based on the biased thought. As a result, the feeling of the client may be easily depressed, or it may be difficult for the client to achieve an objective. It is important to specify an automatic thought causing the feeling of the client, for correcting such a cognitive distortion, or for improving the feeling of the client by allowing the user (client) to find that the user has the distortion. In addition, in the present invention, such information as described below is collectively referred to as "user consciousness information". That is, "user consciousness information" is information of which a user is conscious, and which is necessary to solve a matter about a situation, an automatic thought, a mood, the ground of generating the automatic thought, and the like. In addition, "user consciousness information" is information which becomes a solution such as counterevidence against an automatic thought, or an adaptive thought.

### <Exemplary Embodiment 1>

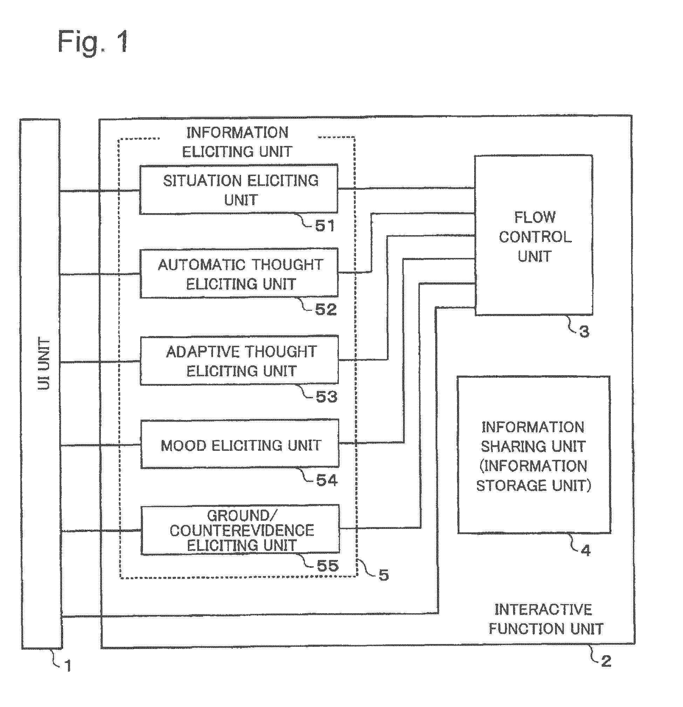

An exemplary embodiment of the present invention will be explained below with reference to the drawings. Fig. 1 is a block diagram illustrating an example configuration of a mental health care system of the first exemplary embodiment of the present invention. The mental health care system illustrated in Fig. 1 includes a user interface unit 1 (hereinafter, UI unit 1) and an interactive function unit 2. In addition, the interactive function unit 2 includes a flow control unit 3, an information sharing unit 4, and information eliciting units 5. In addition, the information eliciting units 5 include a situation eliciting unit 51, an automatic thought eliciting unit 52, an adaptive thought eliciting unit 53, a mood eliciting unit 54, and a ground/counterevidence eliciting unit 55.

The UI unit 1 is a unit has a function of providing a screen, which is a user interface for this system, to a user. In addition, the UI unit 1 executes screen control specified by screen information, in advance. The UI unit 1 is not particularly limited, if the unit 1 includes input/output means such as a display, a mouse, or network communication means such as a LAN card, and is capable of processing conforming to a protocol or a program used for general screen control.

The screen control includes processing of delivering an entered content to the interactive function unit 2 (more specifically, the flow control unit 3 and each information eliciting unit 5) so that, for example, after entry of information by a client, the interactive function unit 2 can present sympathy with the content of the entry, and present a proposal about the content. The phrase "after entry" refers to timing at which, for example, a "next" button or the like on the screen in which the entry is made is pressed down. The phrase "after entry" may also refer to timing at which, for example, a text box falls out of focus. The phrase "after entry" may also refer to timing at which, for example, a return key is pressed down following input of "." (period) in a key input from a user.

In addition, the screen control includes processing of notifying the interactive function unit 2 of information on screen transition or various events on a screen, in order to, for example, enable such interaction that the interactive function unit 2 prompts a client to makes an entry before entry by the client. The phrase "before entry" refers to timing at which, for example, a button for directing screen transition, such as "next", is pressed down, in a screen associated with the step previous to a targeted step. The phrase "before entry" may also refer to timing at which, for example, a text box for entering information or the like falls out of focus in an input screen associated with a targeted step. The phrase "before entry" may also refer to timing at which, for example, a return key is pressed down in a screen associated with the step previous to a targeted step, when input of the return key is pre-specified to the end of input from a user.

In addition, the screen control includes processing of, for example, when a button for requesting interactive support by a client, or the like, is placed on a screen, notifying the interactive function unit 2 of pressing down of the control button or the like. The screen control may also include, for example, such processing as described below in the step of allowing a user to input some characters. That is, the screen control may include, for example, measurement of the number of characters every predetermined time, and a check of the increase of the characters input, for operating a flow switch in a condition that a value obtained by measuring the number of the characters every pre-specified predetermined time is not increased. The screen control may also include processing of providing information on the number of input characters so that the interactive function unit 2 can measure the number of characters every predetermined time and check the increase thereof. In addition, the predetermined time mentioned above, i.e., a measurement interval, may be constant. Such a measurement interval may also be variable, in consideration of requiring some time for beginning of writing.

In addition, all of these types of the screen control are pre-specified by screen information, and the UI unit 1 may execute processing according to a protocol or a program of general screen control.

The flow control unit 3 controls an entire flow. Specifically, the flow control unit 3 directs the information eliciting unit 5 to start or to end processing. In addition, the flow control unit 3 delivers information between steps in the information eliciting units 5, if necessary.

Each processing unit of the information eliciting units 5 (the situation eliciting unit 51, the automatic thought eliciting unit 52, the adaptive thought eliciting unit 53, the mood eliciting unit 54, or the ground/counterevidence eliciting unit 55 in this example) executes each assigned step of processing. Specifically, each processing unit of the information eliciting units 5 allows a user to input information via the UI unit 1, or outputs such a message as to build a therapeutic relationship, in correspondence with a step according to cognitive reframing. As for processing that is common in each processing unit of the information eliciting units 5, a processing unit that carries out the common step may also be separately arranged. Each processing unit may be configured to optionally call up the processing unit that carries out the common step.

Specific step processing will be explained below. Processing executed by the mental health care system of the present exemplary embodiment includes at least an automatic thought eliciting step and an adaptive thought eliciting step. In addition, the processing executed by the mental health care system of the present exemplary embodiment preferably further includes a situation eliciting step in addition to the steps. The processing executed by the mental health care system of the present exemplary embodiment may further include a mood eliciting step and a ground/counterevidence eliciting step. Each of the steps is basically executed in a sequence described below. That is, "situation eliciting step" is executed, if "situation eliciting step" is present. Then, "mood eliciting step" is executed if "mood eliciting step" is present. Or "mood eliciting step" is executed, as necessary, when "mood eliciting step" is present. Then, "automatic thought eliciting step" is executed. Then, "ground/counterevidence eliciting step" is executed if "ground/counterevidence eliciting step" is present. Or "ground/counterevidence eliciting step" is executed, as necessary, when "ground/counterevidence eliciting step" is present. Then, "adaptive thought eliciting step" is executed. The phrase "when a certain step is present" as used herein means "if the step is implemented". In addition, the phrase "as necessary, when certain is present" represents that assuming that the step is implemented, when information to be elicited in the step is obtained in the previous step, the step may be omitted. Further, the phrase "as necessary, when certain is present" represents that such flow control as described below may be executed. That is, a flow control, that the step is temporally omitted and the next step is executed, and if information necessary for the next cannot be elicited, the step omitted is executed, may be performed.

### [Situation Eliciting Step]

First, the situation eliciting step will be explained below. In the present exemplary embodiment, the situation eliciting unit 51 in the information eliciting units 5 is associated with the processing step. The situation eliciting unit 51 executes various types of processing for eliciting "situation" from a client in the situation eliciting step. Information elicited from the client by the situation eliciting unit 51 is specific information on a situation when an automatic thought with a cognitive distortion is generated. The information may be, for example, information on an event that recently a user felt to be unpleasant, or a situation in which a user's feeling has been recently perturbed, or the like. In the step, the situation eliciting unit 51 outputs a message for prompting input of information on the problem situation of the user, and accepts the input of the information (information on the problem situation of the user) from the user.

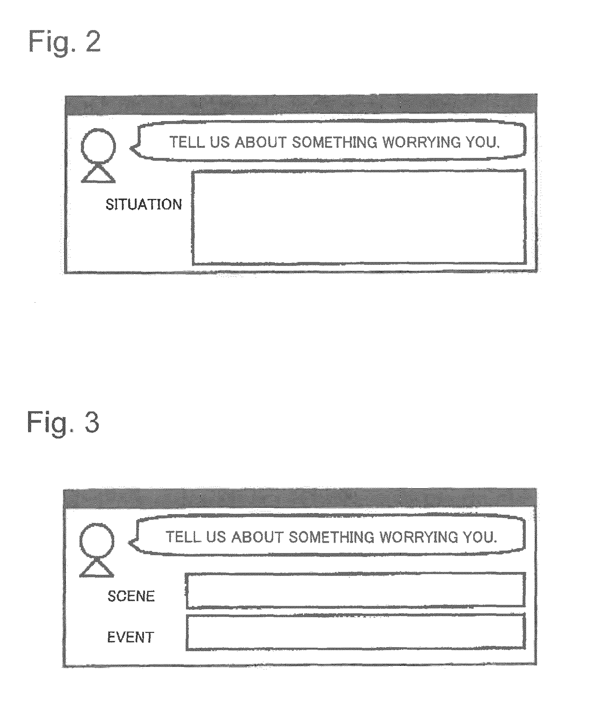

Figs. 2 and 3 are explanatory drawings illustrating examples of screens used in the situation eliciting step.

The screen illustrated in Fig. 2 includes a region in which a message for prompting an entry of information on a situation, such as "Tell us about something worrying you", more specifically, a message for prompting an entry of information on the problem situation of a user, is displayed. The screen illustrated in Fig. 2 also includes an entry field for entering a situation. The situation eliciting unit 51 allows a client to input a text representing "situation" felt by the client, for example, using such a screen as illustrated in Fig. 2, in the situation eliciting step. A screen created beforehand may be utilized as the screen, or a screen may be generated each time. Parts for the screen and screen information that defines the screen control thereof may be prepared beforehand, and the situation eliciting unit 51 may reflect the content of a message or the like to be displayed, in the screen in real time. This point similarly applies to screens used in other processing step.

The screen illustrated in Fig. 3 includes two entry fields for inputting a worried situation separately into "scene" and "event". The situation eliciting unit 51 may elicit a problem situation from a client, for example, using the screen illustrated in Fig. 3. The screen may include an input field for inputting "time" instead of "scene". The situation eliciting unit 51 allows user to conscious of "Slice of Time" easily, by "scene" or "time" and "event" to be separately input. In addition, by reducing the number of rows of each entry (for example, to one row), the effect, that content to be entered by a user is prevented from being discursive, by narrowing the content, is obtained. In addition, a user's intention of organizing content to be written can be affected by downsizing an apparent entry space, for example, by limiting the entry space to a size within not more than the predetermined number of characters (for example, not more than 100 characters, or the like). Only the size of the apparent entry space may be limited, and the number of characters that can be actually input may not be limited. It is effective for enhancing the effect of improving a feeling of the user, by clarifying a scene in such a manner to cause narrowing worries to one.

In both examples illustrated in Fig. 2 and Fig. 3, the messages are output using balloon shapes in order to produce interactive feelings. In addition, in the situation eliciting unit 51, the balloon is disposed at an upper position in the screen and characters are allowed to be relatively enlarged so that a dialog gets into the eyes first. The situation eliciting unit 51 may also slowly display the characters of the message on a one-by-one basis in order to produce an interactive feeling and to attract user's attention.

The situation eliciting unit 51 may also output, as an interactive function, a message presenting sympathy with a worried situation of a client, or an event occurred in the client. A place in which the message is displayed may be on the screen of the step, or may be on the screen of the next step. In other words, the situation eliciting unit 51 specifies the situation annoying the client, or the event causing the client to languish, based on an input content. And the situation eliciting unit 51 outputs a sympathetic message. As a result, the situation eliciting unit 51 can build a therapeutic relationship (trust relationship) which is important for mental health care between the client and the system.

The situation eliciting unit 51 may handle, as input, text information written in a situation entry field by a client, and may generate and output a sympathetic message. When a situation is written separately in "scene" or "time" and "event", the situation eliciting unit 51 may handle only the information of "event" as input.

The sympathetic message preferably has content in which summarization of a client's feeling of languishing is included. The term "summarization" means the summary and repeat of what happened to client or the representation and repeat of the feeling of the client. The situation eliciting unit 51 may generate a message presenting all these contents.

For example, it is assumed that the phrase " 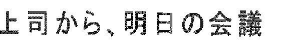 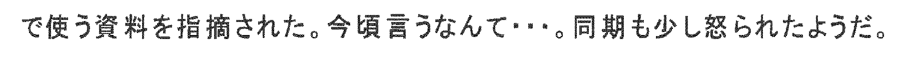 (A document used in a tomorrow's meeting was pointed out by my boss. He told me that now, after all this time... My contemporary also seemed to be blamed.)", is input. In this case, the situation eliciting unit 51 may generate and output the sympathetic messages such as "The document was pointed out, wasn't it?" and "You were sad about it, weren't you?". In such a manner, the situation eliciting unit 51 may specify a point causing a person (user) to feel languishing, i.e., "worry point", and a feeling of the user, based on a document, in which a problem situation for the user is described. And the situation eliciting unit 51 may output a message in which the worry point and the feeling are repeated. In this case, the situation eliciting unit 51 may estimate the feeling of the person and includes the estimated feeling into a sympathetic message, even if the feeling is not specified.

The worry point and the feeling may be specified by, for example, extracting a phrase representing the worry (hereinafter referred to as "worry term") and a phrase representing the feeling (hereinafter referred to as "feeling term") using a dictionary. In this case, it is important that the extracted phrases refer an event occurred with regard to the client himself/herself and the feeling of the client himself/herself.

The situation eliciting unit 51 may execute various individual determinations such as self/others determination, negative determination, interrogative determination, conjectural determination, and conditional determination, for example, based on expressions arranged at before and behind the worry term and the feeling term extracted by using the dictionary. In addition, the situation eliciting unit 51 may determine, by synthesizing the results, whether or not the extracted worry term represents the matter (situation) occurred in a client and whether or not the extracted feeling term represents the feeling felt by the client. For a processing procedure, the extraction of the worry term and the feeling term, the various individual determinations, and the integrated determination may be executed in this sequence. All the individual determinations are not essential. Individual determinations considered to be needed may be appropriately adopted depending on the kind of handled cognition, the kind of a targeted user, or the like.

A more specific explanation will be given below. The extraction of the worry term and the feeling term is carried out, for example, in such a manner as described below. That is, the situation eliciting unit 51 registers, in the dictionary, the original forms of terms in expressions that easily become worry terms or feeling terms, in advance. The situation eliciting unit 51 matches the original forms of terms obtained by carrying out morphological analysis or the like of an input sentence, with the dictionary, to thereby extract a worry term or a feeling term.

For example, it is assumed that " 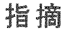 (a verb represents "point out")- 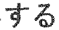 (an auxiliary verb )- 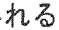 (: an auxiliary verb representing passive voice)" and " 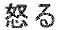 (a verb represents "blame")- 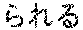 (an auxiliary verb representing passive voice)" are registered in the worry term dictionary. In this case, it is assumed that " 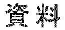 (a noun represents "document")/ 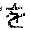 (a case particle)/ 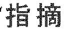 (a verb represents "point out")/ 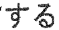 (an auxiliary verb)/ 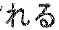 (an auxiliary verb representing passive voice)" is obtained as the result of morphological analysis of the input sentence of " 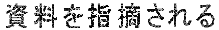 (A document is pointed out.)" In this case, the situation eliciting unit 51 may extract, as a worry term, "pointed out" which is a portion corresponding to " 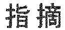 (a verb represents "point out")/ 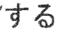 (an auxiliary verb )/ 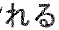 (an auxiliary verb representing passive voice)", by matching with the dictionary described above.

The various kinds of determination processing of extracted worry terms and feeling terms will be explained below. First, the self/others determination processing will be explained. For example, it is assumed that the expressions of " 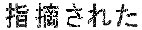 (a verb represents "pointed out")" and " 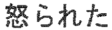 (a verb represents "blamed")" are extracted as worry terms from the sentence of " 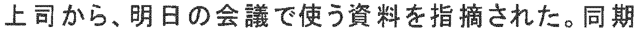 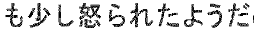 (A document used in a tomorrow's meeting was pointed out by my boss. My contemporary also seemed to be blamed.)" In the example mentioned above, " 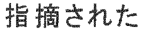 (a verb represents "pointed out")" is an event occurred in the person concerned(self), and " 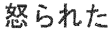 (a verb represents "blamed")" is an event occurred in the contemporary(another person). Thus, by determining whether a subject of the sentence is the person concerned or another person, the situation eliciting unit 51 can preferentially process the expression in which the person concerned is the subject.

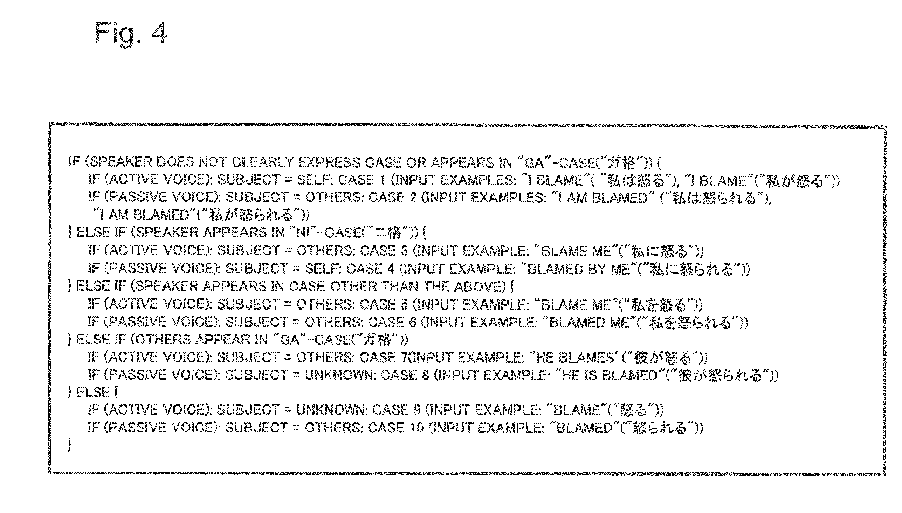

Fig. 4 is an explanatory drawing illustrating an example of the flow of self/others determination processing in Japanese. As illustrated in Fig. 4, based on a worry term or feeling term extracted in an input text and on expressions arranged at before and behind of the worry term or feeling term, the situation eliciting unit 51 may determine the subject of one sentence including the extracted worry term or feeling term. Specifically, the situation eliciting unit 51 may determine, based on the extracted expression described above, whether the subject of the sentence (sentence including feeling expression or event expression) is a self or another person, or unknown. In the example illustrated in Fig. 4, the situation eliciting unit 51 determines the kind of a case (genitive case) in which a speaker appears. The situation eliciting unit 51 further combines the case of the speaker appeared with the voice of a verb in the sentence which includes the extracted worry term or feeling term, to determine the subject.

The negative determination processing will be explained below. In the negative determination processing, the situation eliciting unit 51 determines whether an extracted worry term or feeling term is negated or not. If the determination result is "negative", the situation eliciting unit 51 considers that the term is not a worry point. In addition, the situation eliciting unit 51 excludes the worry term or the feeling term from targets to be subjected to determination whether or not each of the targets is worry or a feeling generated in a client. Alternatively, the situation eliciting unit 51 carries out processing of decreasing the level that indicates whether or not the worry term or the feeling term is worry or a feeling generated in the client. For example, it is assumed that a case where the portion of " 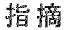 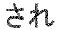 ( a verb representing "pointed out")" is extracted as a worry term from the input sentence of " 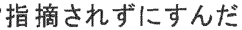 (need not be pointed out)". In this case, the situation eliciting unit 51 may count phrases representing negatives, such as "not" and "impossible", or expressions used for representing negatives (hereinafter collectively referred to as "negative expression"), appearing behind the extracted worry term or feeling term. If the counted number is an odd number, the situation eliciting unit 51 may determine that the worry term or the feeling term is negated.

The interrogative determination processing will be explained below. In the interrogative determination processing, the situation eliciting unit 51 determines whether a sentence including a worry term or a feeling term is an interrogative sentence or not. If the sentence is an interrogative sentence, the situation eliciting unit 51 considers that the sentence does not represent a matter/feeling that has actually occurred. In addition, the situation eliciting unit 51 excludes the worry term or the feeling term from a target to be subjected to determination whether or not each of the targets is worry or a feeling generated in a client. Alternatively, the situation eliciting unit 51 carries out processing of decreasing the level that indicates whether or not the worry term or the feeling term is worry or a feeling generated in the client. For example, it is assumed that a case where the portion of " 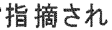 (a verb represents "pointed out")" is extracted as a worry term from the expression of " 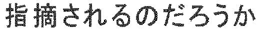 (Is ... pointed out?)". In this case, if a mark or an expression used in interrogative representation, such as " 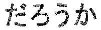 (Is ... ?)", " 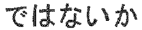 (isn't it?)", or a mark "?", (hereinafter collectively referred to as "interrogative expression") is present behind the extracted worry term or feeling term, the situation eliciting unit 51 may determine that the sentence is an interrogative sentence.

The conditional determination processing will be explained below. In the conditional determination processing, the situation eliciting unit 51 determines whether a worry term or a feeling term is a conditional term or not. If the extracted worry term or feeling term is a conditional term, the situation eliciting unit 51 considers that it does not represent a matter/feeling that has actually occurred. In addition, the situation eliciting unit 51 may carry out processing of excluding the worry term or the feeling term from a target to be subjected to determination whether or not each of the targets is worry or a feeling generated in a client, or may carry out processing of decreasing the priority. For example, it is assumed that a case where the portion of " 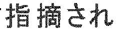 (a verb represents "pointed out")" is extracted as a worry term from the expression of " 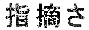 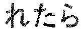 (If ... pointed out)". In this case, if a term representing a conditional clause, such as " 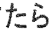 (if)" or " 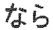 (when)", is present in a character string behind the extracted worry term or feeling term, the situation eliciting unit 51 may determine that the term is a conditional term.

The conjectural determination processing will be explained below. In the conjectural determination processing, the situation eliciting unit 51 determines whether a worry term or a feeling term is conjectural or not. If the worry term or the feeling term is conjectural, the situation eliciting unit 51 considers that it does not represent a matter/feeling that has actually occurred. In addition, the situation eliciting unit 51 may carry out processing of excluding the worry term or the feeling term from a target to be subjected to determination whether or not each of the targets is worry or a feeling generated in a client. The situation eliciting unit 51 may also carry out processing of decreasing the level indicating whether or not the worry term or the feeling term is worry or a feeling generated in the client. For example, it is assumed that a case where the portion of " 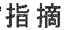 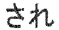 (a verb represents "pointed out")" is extracted as a worry term from the expression of " 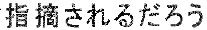 (will be pointed out)". In this case, if a phrase representing conjecture or an expression used when conjecture is represented, such as " 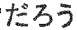 (will)" or " 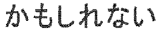 (may)", (hereinafter collectively referred to as "conjectural expression"), is present in a character string behind the extracted worry term or feeling term, the situation eliciting unit 51 may determine that the term is conjectural.

The integrated determination processing will be explained below. In the integrated determination processing, the situation eliciting unit 51, as necessary, carries out determination, such as self/others determination, negative determination, interrogative determination, conditional determination, or conjectural determination, of, for example, a worry term or a feeling term detected utilizing each determination processing as described above. In addition, the situation eliciting unit 51 may generate a vector including these elements (for example, five self/others, negative, interrogative, conditional, and conjectural elements) based on the results of those determination. By assuming, that the inner product of the generated vector and a vector including, as an element, an importance degree defined for each element, as an evaluation value, the situation eliciting unit 51 may calculate the evaluation values of all worry terms and feeling terms in an input sentence. Such an evaluation value may be normalized with a value such as the total sum of importance degrees. The situation eliciting unit 51 may apply intensities representing the worry magnitudes and feeling magnitudes of worry terms and feeling terms into a dictionary or the like, and the situation eliciting unit 51 may consider the seriousness of worry to the evaluation values, by, e.g., multiplying the calculated evaluation values with the intensities. Finally, the situation eliciting unit 51 determines terms with the highest evaluation value, or terms with several top values as worry terms and feeling terms for a client.

In the calculation of the evaluation values, the situation eliciting unit 51 may carry out individual selection, such that, e.g., it is essential that a subject is "self", or the subject is excluded from targets if the subject is "other".

In addition to these methods, a person may determine whether or not a sentence including "worry term" or "feeling term", or the like to be targeted (for example, a sentence including a phrase or an expression that can be a worry term or a feeling term registered beforehand) represents a matter that has actually occurred in a user. In addition, this system may allow a machine learning module to execute learning using the result as a positive example or a negative example. The situation eliciting unit 51 may carry out the determinations described above by using the machine learning module that has executed such learning.

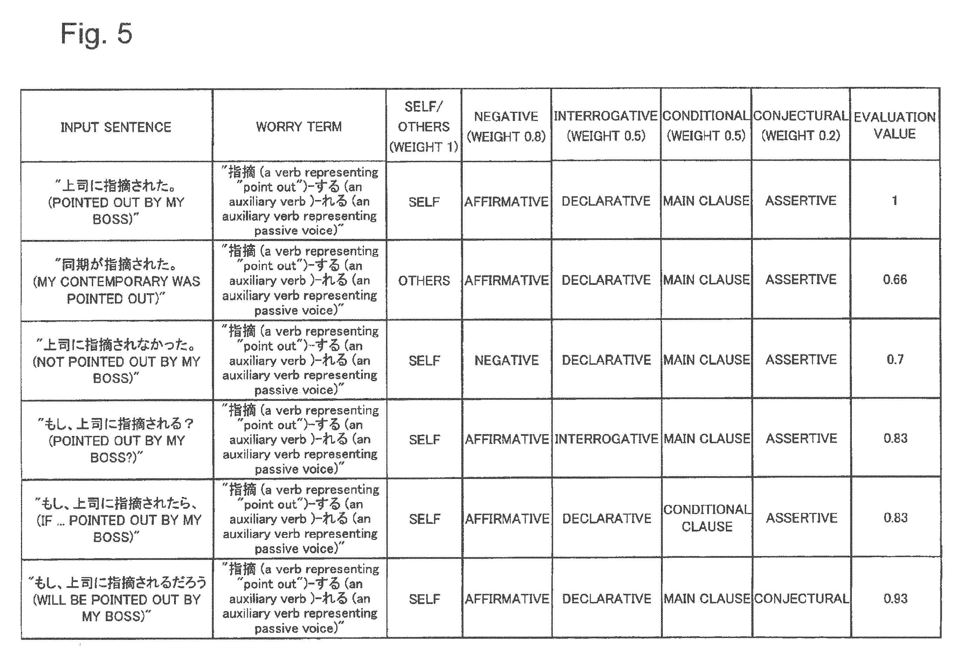

Fig. 5 illustrates examples of integrated determination results together with examples of input sentences. In the examples illustrated in Fig. 5, the result of each determination processing, and an evaluation value which is the result of integrated determination processing, are listed with regard to each worry term extracted from each of the six input sentences. In the examples illustrated in Fig. 5, the situation eliciting unit 51 generates a vector including such elements as described below. That is, such a vector includes an element that is 1 if the result of the self/others determination processing is "self", or 0 if the result is "other" or "unknown". In addition, such a vector includes an element that is 1 if the result of the negative determination processing is "affirmative sentence" or 0 if the result is "negative sentence". In addition, such a vector includes an element that is 1 if the result of the interrogative determination processing is "declarative sentence" or 0 if the result is "interrogative sentence". In addition, such a vector includes an element that is 1 if the result of the conditional determination processing is "main clause" or 0 if the result is "conditional clause". In addition, such a vector includes an element that is 1 if the result of the conjectural determination processing is "assertive" or 0 if the result is "conjectural". Further, the situation eliciting unit 51 generates a weight vector including such elements as described below. That is, such a weight vector includes the importance degree of the self/others determination ("1" in the examples), the importance degree of the negative determination ("0.8" in the examples), the importance degree of the interrogative determination ("0.5" in the examples), the importance degree of the conditional determination ("0.5" in the examples), and the importance degree of the conjectural determination ("0.2" in the examples) as the elements. The situation eliciting unit 51 adopts, as the evaluation value, the result obtained by calculating the inner product of the two generated vectors.

In the examples illustrated in Fig. 5, the evaluation value of a worry term " 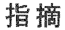 (a verb representing "point out")- 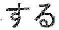 (an auxiliary verb)- 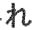 - 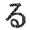 (an auxiliary verb representing passive voice)" ( 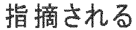 (pointed out)) extracted from the input sentence of " 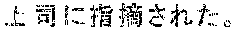 (pointed out by my boss)" is "1". In addition, the evaluation value of a worry term " 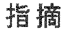 (a verb representing "point out")- 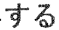 (an auxiliary verb)- 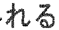 (an auxiliary verb representing passive voice)" extracted from the input sentence of " 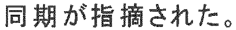 (my contemporary was pointed out)" is "0.66". In addition, the evaluation value of a worry term " 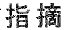 (a verb representing "point out")- 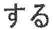 (an auxiliary verb)- 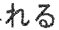 (an auxiliary verb representing passive voice)" extracted from the input sentence of " 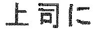 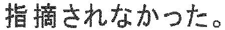 (not pointed out by my boss)" is "0.7". In addition, the evaluation value of a worry term " 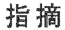 (a verb representing "point out")- 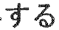 (an auxiliary verb )- 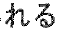 (an auxiliary verb representing passive voice)" extracted from the input sentence of " 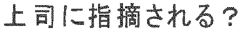 (pointed out by my boss?)" is "0.83". In addition, the evaluation value of a worry term " 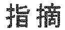 (a verb representing "point out")- 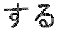 (an auxiliary verb )- 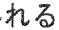 (an auxiliary verb representing passive voice)" extracted from the input sentence of " 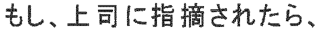 (If ... pointed out by my boss)" is "0.83". In addition, the evaluation value of a worry term " 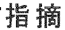 (a verb representing "point out")- 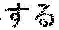 (an auxiliary verb )- 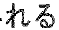 (an auxiliary verb representing passive voice)" extracted from the input sentence of " 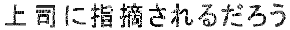 (will be pointed out by my boss)" is "0.93".

If the six example sentences described above are written by one client, it can be judged that the term " 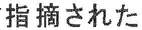 (pointed out)" which is a worry term extracted from the sentence " 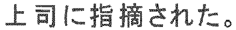 (pointed out by my boss)" with the highest evaluation value has most likely occurred in the client. The situation eliciting unit 51 can narrow down worry terms and feeling terms to one or a predetermined number, by carrying out the integrated determination processing on all the worry and feeling terms in an input sentence, rather than on only the same worry term.

In such a manner, the situation eliciting unit 51 specifies a point representing a matter causing a person concerned (client) to feel languishing, i.e., a worry point, and, if possible, a feeling, from a document in which a problem situation is described. When the worry point and the feeling are specified, the situation eliciting unit 51 generates and outputs a sympathetic message based on the specified worry point and feeling.

For example, it is assumed that the sentence of "5 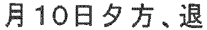 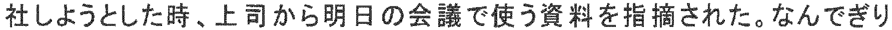 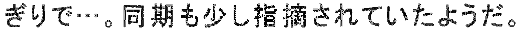 (When I was going to leave work in the evening of May 10, a document to be used in a tomorrow's meeting was pointed out by my boss. Why at the time of this last minute. My contemporary also seemed to have been somewhat pointed out.)" is input into the system. In addition, it is assumed that the two points of " 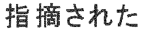 (pointed out)" and " 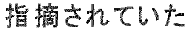 (have been pointed out)" in an input sentence are extracted, and the former point of them is specified as a worry point, by the processing of specifying a worry term.

In the processing of generating a sympathetic message, first, the situation eliciting unit 51 carries out acquisition of " 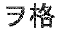 ('wo'-case: particle representing objective)" or " 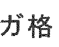 ("ga"-case: particle representing nominative)" from a clause before the specified worry point or feeling, or the like, and formats a phrase including the extracted worry term or feeling term. In this example, " 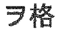 ('wo'-case: particle representing objective)" of " 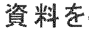 (a document)" is acquired from the clause before " 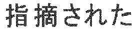 (pointed out)", which is a worry point, and is synthesized with the character string representing the worry term, to thereby obtain the formatted phrase of " 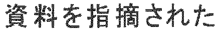 (a document was pointed out)". The situation eliciting unit 51 generates a sympathetic message using the obtained formatted phrase. In this example, the situation eliciting unit 51 may generate a message such as "The document was pointed out, wasn't it? You languished in it, didn't you?". In this case, the portion of "wasn't it? You languished in it, didn't you?" may be prepared as a template. The situation eliciting unit 51 may select a template sentence in a predetermined sequence or on a random basis from a plurality of kinds of template sentences that are prepared in advance. As a result, a different message is presented to even a user who uses the system several times, whenever the user uses the system. As a result, it may be avoided that the user gets tired of the system.

In a case in which a feeling can be acquired from the input sentence, the situation eliciting unit 51 may replace the portion of "languished" with the feeling term that can be acquired. The situation eliciting unit 51 may associates worry terms with feelings that are easily evoked in association with worry terms, in the dictionary in advance, and may estimate a feeling from a worry term even if any feeling term is not included in an input sentence. The situation eliciting unit 51 may also be capable of generating a sympathetic message including an expression representing the estimated feeling.

In addition, the situation eliciting unit 51 may allow information (such as an input sentence, a summary of a matter occurring in a user, a worry term, or a feeling term) obtained in this step processing to be stored in the information sharing unit 4 and may enable the information to be referred in the other steps.

### [Automatic Thought Eliciting Step]

The automatic thought eliciting step will be explained below. In the present exemplary embodiment, the automatic thought eliciting unit 52 in the information eliciting unit 5 is associated with the step processing. The automatic thought eliciting unit 52 executes various types of processing for eliciting "automatic thought" from a client, in the automatic thought eliciting step. Information elicited from the client by the automatic thought eliciting unit 52 is, specifically information representing a thought generated in the client in the situation obtained by questioning in the situation eliciting step. The information elicited from the client by the automatic thought eliciting unit 52 is particularly information representing a thought leading to a languishing feeling. In the step, the automatic thought eliciting unit 52 outputs a message for prompting input of an automatic thought generated in a situation in which a user's feeling is perturbed. Then, the automatic thought eliciting unit 52 accepts the input of the information (information representing the automatic thought) from the user.

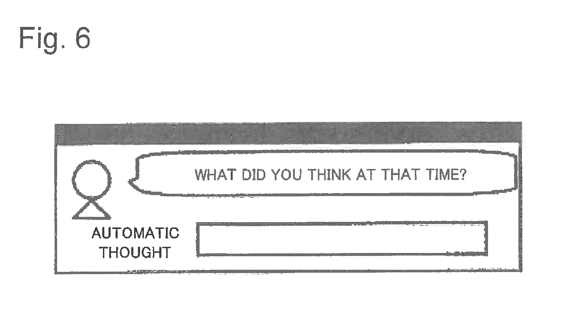

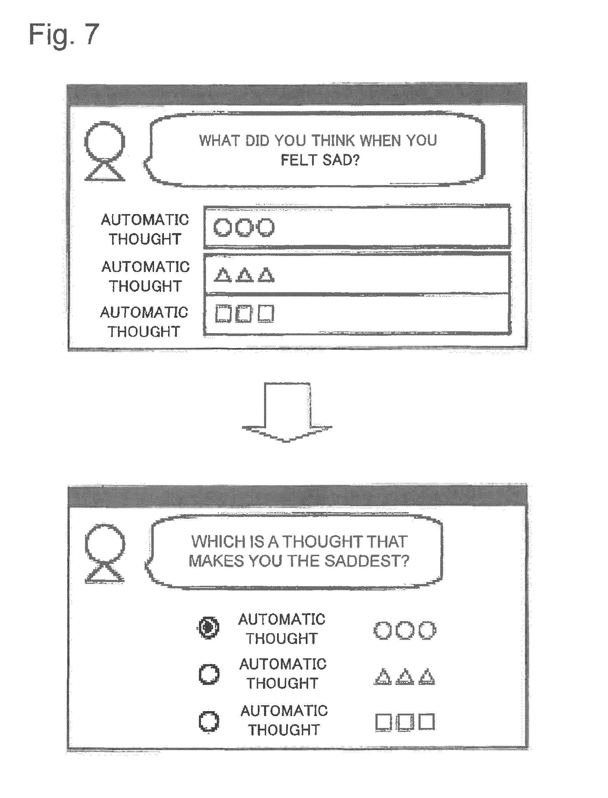

Fig. 6 and Fig. 7 are explanatory drawings illustrating examples of screens used in the automatic thought eliciting step. The automatic thought eliciting unit 52 allows a client to input a text representing "automatic thought" felt by the client, for example, using such a screen as illustrated in Fig. 6 in the automatic thought eliciting step. Such a screen includes a region, in which the message for prompting the entry (input) of the automatic thought such as "What did you think at that time?" is displayed, and an entry field for an automatic thought.

The automatic thought eliciting unit 52 may also use a screen including a message for prompting entry of an automatic thought, for example, as illustrated in Fig. 7. The message illustrated in Fig. 7 is a message including "feeling of client" acquired in any of the previous steps. In such a manner, the automatic thought eliciting unit 52 can allow a user to easily write an automatic thought leading to a languishing feeling.

In the examples illustrated in Fig. 6 and Fig. 7, the messages are also output using balloon shapes in order to produce interactive feelings. In addition, the balloons are disposed at upper positions in the upper screens and characters are relatively enlarged so that first, a dialog gets into the eyes.

The automatic thought eliciting unit 52 may also allow a client to write a plurality of automatic thoughts using a screen including a plurality of entry fields, in order to allow as many automatic thoughts as possible to be mentioned, for example, as illustrated in Fig. 7. Then, the automatic thought eliciting unit 52 may allow the client to narrow the automatic thoughts to one, by using a screen for selecting the automatic thought leading to the most languishing feeling, from the entered automatic thoughts. In such a manner, the automatic thought eliciting unit 52 can allow the client to easily objectively reflect himself or herself by using the screen for mentioning as many automatic thoughts as possible and the screen for selecting the automatic thought. The automatic thought eliciting unit 52 may also output a message such as "Which is a thought that makes you the saddest?" in the screen for selecting one automatic thought from a plurality of automatic thoughts. The automatic thought eliciting unit 52 may also output a message, for prompting selection of an automatic thought including "feeling of client" acquired in any of the previous steps, in such a screen.

The automatic thought eliciting unit 52 may estimate a feeling in this step when it is impossible to specify a feeling in the situation eliciting step or when the processing of estimating a feeling is not carried out. The automatic thought is preferably associated with a languishing feeling felt by a client. If the situation of the client can be known, a feeling presumed to have been felt by the client in the situation can be inferred to some extent. Thus, the automatic thought eliciting unit 52 may estimate a feeling based on information representing the situation obtained in the situation eliciting step. By outputting the estimated feeling together with a questioning message for obtaining an automatic thought, the automatic thought eliciting unit 52 can allow user easily to reveal the automatic thought leading to the feeling.

When the system simply questions a client on a his or her thought without any leading, the client often writes a feeling such as "I was sad." for a question for the automatic thought, such as "What kind of thought do you generate at that time?". It is effective to allow a question message to pre-include a feeling in order to avoid such a reply. This is because, for the question message including the feeling, there are a few clients who write the same feeling included in the message. By the message, for prompting entry of an automatic thought, in which the feeling at that time is included in such a manner, the automatic thought eliciting unit 52 can prevent the client to enter a feeling in an entry field for writing an automatic thought.

For example, assuming that text information written in an entry field for a situation in the situation eliciting step by a client is input, the automatic thought eliciting unit 52 may estimate a feeling based on a phrase included in the input text, as processing of estimating a feeling. The automatic thought eliciting unit 52 may also output a message including the estimated feeling, for prompting entry of an automatic thought. The automatic thought eliciting unit 52 may also output an introduction message before the message for prompting entry of an automatic thought.

For example, the automatic thought eliciting unit 52 may output a sentence such as "You were sad about it, weren't you? What kind of thought did you have at that time?", for the input sentence of " 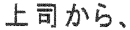 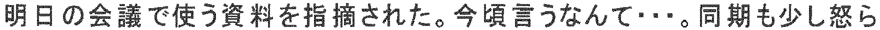 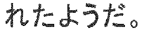 (A document to be used in a tomorrow's meeting was pointed out by my boss. He told me that now, after all this time. My contemporary also seemed to be blamed.)".

In this processing, the automatic thought eliciting unit 52 may utilize a prepared template, if the automatic thought eliciting unit 52 may specify the feeling that the client felt languishing from a document in which a problem situation is described. The processing of estimating a feeling from information on an event may be similar to the method explained in the situation eliciting step. As also mentioned in processing of specifying a worry point and a feeling in the situation eliciting step, it is important to extract or estimate a matter actually felt by the client in the extraction and estimation of a feeling.

When a dictionary in which worry terms are associated with feelings is utilized in the estimation of a feeling, categories of which the number is predetermined, such as "sad", "anxiety", and "anger", (hereinafter referred to as "feeling categories") may be set for the feelings. For each worry term, one or more feeling categories evoked by the term may be stored together with the association degree of the term in such a dictionary.

When the feeling categories are utilized in the estimation of a feeling, the automatic thought eliciting unit 52 may acquire feeling categories associated with all worry terms specified as the worry term being generated in the client himself of herself. For each acquired feeling category, the automatic thought eliciting unit 52 may calculate, as a certainty factor, the linear sum of the association degree of the category and the evaluation value of the associated worry term. Then, the automatic thought eliciting unit 52 outputs a feeling category with the highest certainty factor as the feeling of the client.

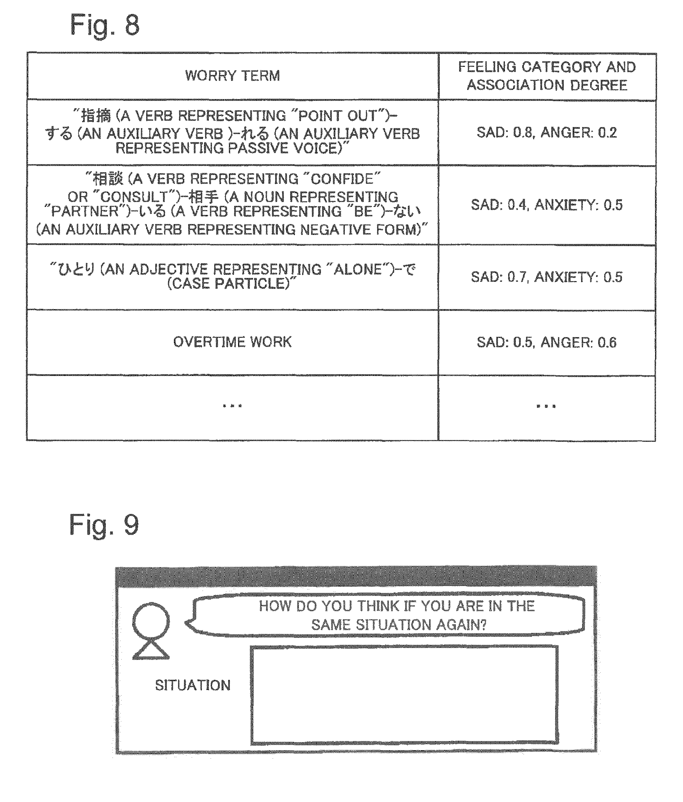

Fig. 8 is an explanatory drawing illustrating an example of a dictionary used in estimation of a feeling. As illustrated in Fig. 8, with regard to a term of an expression that likely becomes a worry term, the original form of the term of the expression may be pre-registered together with the degree of association with a feeling category in a dictionary. In Fig. 8, for example, in association with the original form " 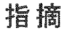 (a verb representing "point out")- 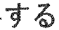 (an auxiliary verb )- 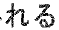 (an auxiliary verb representing passive voice)" of a worry term such as " 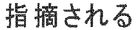 (pointed out)", the degree of the association of the original form with a feeling category "sad" of "0.8" and the degree of the association of the original form with a feeling category "anger" of "0.2" are registered. In addition, for example, in association with the original form " 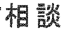 (a verb representing "confide" or "consult")- 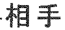 (a noun representing "partner")- 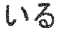 (a verb representing "be")- 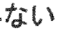 (an auxiliary verb representing negative form)" of a worry term such as " 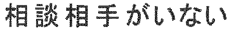 (I have no confidant (consulting partner).)", the degree of the association of the original form with the feeling category "sad" of "0.4" and the degree of the association of the original form with a feeling category "anxiety" of "0.5" are registered. In addition, for example, in association with the original form " 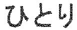 (an adjective representing "alone")- 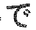 (case particle)" of a worry term such as " 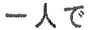 (by oneself)", " 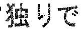 (alone)", or " 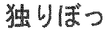 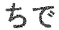 (lonely)", the degree of the association of the original form with the feeling category "sad" of "0.7" and the degree of the association of the original form with the feeling category "anxiety" of "0.5" are registered. In this example, it is assumed that a term such as " 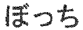 (only)" may be included between phrases joined to each other by "-" as illustrated in Fig. 8. In addition, in association with the original form " 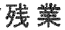 (overtime work)" of the worry term of " 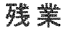 (overtime work)", the degree of the association of the original form with the feeling category "sad" of "0.5" and the degree of the association of the original form with the feeling category "anger" of "0.6" are registered.

In this case, it is assumed that the sentence of " 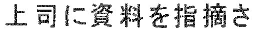 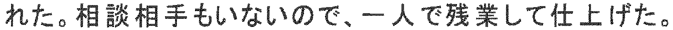 (A document was pointed out by my boss. Because I have no confidant, I worked overtime to finish it by myself.)" is input as text information representing a situation. In addition, it is assumed that " 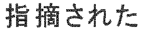 (pointed out)", " 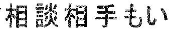 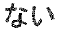 (have no confidant)", " 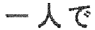 (by oneself)", and " 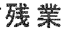 (overtime work)" are specified as worry terms included in an input sentence in the processing of specifying a worry point. It is also assumed that the evaluation values of " 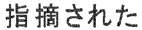 (pointed out)", " 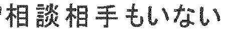 (have no confidant)", " 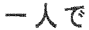 (by oneself)", and " 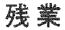 (overtime work)" are 1, 1, 0.8, and 0.5, respectively. In this case, the automatic thought eliciting unit 52 may acquire a feeling category associated with each specified worry term, and calculates a certainty factor for each feeling category from the product sum of an association degree and an evaluation value, as described below.

In other words, the certainty factor of the feeling category "sad" is calculated as "0.8×1+0.4×1+0.7×0.8+0.5×0.5=2.01" based on the calculation results of the dictionary illustrated in Fig. 8 and the above-mentioned evaluation values. In addition, the certainty factor of the feeling category "anxiety" is calculated as "0.5×1+0.5×0.8=0.9" based on the calculation results of the dictionary illustrated in Fig. 8 and the above-mentioned evaluation values. In addition, the certainty factor of the feeling category "anger" is calculated as "0.2×1+0.6×0.5=0.5" based on the calculation results of the dictionary illustrated in Fig. 8 and the above-mentioned evaluation values. Based on the above results, the automatic thought eliciting unit 52 may regard the feeling category "sad" with the highest certainty factor as the estimated result. The automatic thought eliciting unit 52 may generates a message including the term "sad" by, e.g., applying the term "sad" to a template prepared beforehand. In this example, the automatic thought eliciting unit 52 may generate a sympathetic message such as "You become sad, don't you?" and add the sympathetic message before a message for prompting entry of an automatic thought. The automatic thought eliciting unit 52 may also generate and output a message for specifying a generated feeling and prompting entry of an automatic thought at that time, such as "What did you think when you felt sad?".

The automatic thought eliciting unit 52 may also include the function of calling for attention of a vague expression included in the expression text of the obtained automatic thought. In cognitive behavior therapy, it is important that a client is conscious of his or her automatic thought. However, a client unfamiliar with this method often expresses an automatic thought as a vague expression such as "may have been". Thus, the automatic thought eliciting unit 52 may detect a vague expression from an input text in the automatic thought eliciting step, and calls for attention for describing an automatic thought in definite form.

The automatic thought eliciting unit 52 may carry out processing of extracting a vague description from text information, as an input, written in an entry field for an automatic thought by a client, as the processing of calling for a vague expression, for example. The automatic thought eliciting unit 52 may also output a message including indication about a method for writing an automatic thought, i.e., a way of expressing the automatic thought, when there is a vague description.

For example, the automatic thought eliciting unit 52 may output a sentence such as "A thought 'fail in document creation again' may cause you to languish. It is considered that definite speaking is also good for you." for the input sentence of "I may fail in document creation again."

In the processing of calling for attention of a vague expression, the automatic thought eliciting unit 52 may extract, for example, interrogative expressions and conjectural expressions. The automatic thought eliciting unit 52 may extract the interrogative expressions and conjectural expressions utilizing the interrogative determination and conjectural determination explained in the processing of specifying a worry point or a feeling. Specifically, the automatic thought eliciting unit 52 may carry out the morphological analysis and syntactic analysis of an input sentence and may scan each phrase from the rear of the sentence to find a verb. In addition, the automatic thought eliciting unit 52 may determine whether a conjectural expression such as " 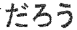 (will)" or " 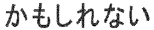 (may)" or an interrogative expression such as " 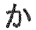 (Is ... ?)" or "?" is present or not in words modified by the verb up to finding of the verb. As a result of the determination, if such an expression is present, the automatic thought eliciting unit 52 may cut out a bundle of a sentence including the characters of the expression from the input sentence then, the automatic thought eliciting unit 52 may correct the end of the bundle of the sentence so that the the sentence becomes in definite form, and may output the corrected sentence as a proposal for correction.

The automatic thought eliciting unit 52 may also include the function of detecting a contradiction between a feeling and an automatic thought. For example, it is assumed that a text representing the automatic thought of a client, or an automatic thought category in which the automatic thought of the client is classified into a predetermined category is input into the automatic thought eliciting unit 52. Further, it is assumed that a text representing the feeling of the client in a case in which the automatic thought is generated, or a feeling category in which the feeling of the client is classified into a predetermined category is input into the automatic thought eliciting unit 52. In this case, the automatic thought eliciting unit 52 may determine the presence or absence of contradiction between the automatic thought and the feeling based on the text representing the automatic thought or the automatic thought category and on the text representing the feeling or the feeling category.

It is considered that an automatic thought relates to four cognitions of "loss", "danger", "unfairness", and "acquisition". Of these, negative automatic thoughts are three cognitions of "loss", "danger", and "unfairness", which are considered to evoke the feelings of "depression", "anxiety", and "anger", respectively. As described above, it is considered that a one-to-one relationship between an automatic thought and a feeling is established when each is classified into a category. In other words, it is considered that "loss" evokes "depression", "danger" evokes "anxiety", and "unfairness" evokes "anger". It is considered that "acquisition" evokes "pleasure". In this example, a category into which an automatic thought is classified with regard to the cognitions described above is referred to as "automatic thought category", while the category of a feeling corresponding to the automatic thought category is referred to as "feeling category".

The automatic thought eliciting unit 52 may estimate a feeling category corresponding to an automatic thought based on, for example, the text expression of the automatic thought or an automatic thought category. In addition, the automatic thought eliciting unit 52 may determine the presence or absence of contradiction between the automatic thought and a feeling, by determining whether or not the estimated feeling category corresponding to the automatic thought and a feeling category into which the feeling input by a client is classified matches with each other.

The automatic thought eliciting unit 52 may use a classifier (model) such as a probability model as a document classification technology, for example, for estimation of a feeling category. Specifically, the automatic thought eliciting unit 52 can use a document classifier such as a generation model or an identification model. The classifier is not particularly limited as long as the model the classifier is capable of specifying a feeling category or an automatic thought category, to which an automatic thought is classified. Any convenient model may be selected depending on a utilization scene. Examples of the generation model include Naive Bayes (simple Bayes classifier), Support Vector Machine (SVM), and the like.

Each cognition category which is an automatic thought category has the characteristic tendency of the way of thinking thereof. The cognition classified into the "loss" category includes many ways of thinking, such as "I am useless.", "Other people regard me as useless.", and "My future is hopeless.", for example, like the expression of "I don't have the bright future." In addition, the cognition classified into the "danger" category includes many ways of thinking, such as "A danger is posed to me.", "I have no ability.", and "I am not supported by others.", for example, like the expression of "A patient with depression surely fails to return to work." In addition, the cognition classified into the "unfairness" category includes many ways of thinking, such as "Although I think I have no problem, other people regard me as useless.", for example, like the expression of "The company deprives me of my freedom." In category classification of an automatic thought, the automatic thought eliciting unit 52 may learn differences between the tendencies of such ways of thinking in a generator.

When it is impossible to acquire sufficient precision only by the characteristic of a word, the automatic thought eliciting unit 52 may carry out learning by adding characteristics specific to an cognitive expression, specifically, a tense, a subject, the voice of a verb, and the type of a sentence to parameters. For example, an automatic thought expression classified into the "loss" category has such characteristics as described below. That is, the automatic thought expression is often represented in the past or present tense. In addition, the automatic thought expression is often represented in the active voice in which the self is the subject. In addition, the automatic thought expression is often represented in question form. For example, an automatic thought expression classified into the "danger" category has such characteristics as described below. That is, the automatic thought expression is often represented in the present or future tense. In addition, the automatic thought expression is often represented in the active voice in which another person is the subject. In addition, the automatic thought expression is often represented in conjectural form. For example, an automatic thought expression classified into the "unfairness" category has such characteristics as described below. That is, the automatic thought expression is often represented in the present or past tense. In addition, the automatic thought expression is often represented in the active voice in which another person is the subject or in the passive voice in which the self is the subject.

It is assumed that there is a contradiction between a feeling elicited from a client (the feeling is input by the client) in the situation eliciting step or a feeling eliciting step mentioned later, and an automatic thought elicited from the client (the feeling is input by the client) in the automatic thought eliciting step, as described above. In this case, the automatic thought eliciting unit 52 may output a message for making a notification that there is the contradiction. In this case, the automatic thought eliciting unit 52 may output a message such as, for example, "Don't you think that evoking of the feeling of '...' due to the thought of '...' is irrational?" or "It is considered that the thought of '...' often makes a person comparatively sad. Why do you feel anger?". In this case, the automatic thought eliciting unit 52 may also output a message for reconfirming or delving into an automatic thought or a feeling.

For example, it is assumed that the sentence of "I always fail in document creation." is input as an automatic thought expression and a feeling term corresponding to the "anxiety" category is input as a term representing a feeling. The automatic thought category that most matches with the worry term of "fail in" is "depression". In this case, the automatic thought eliciting unit 52 may output a message for reconsideration of an automatic thought, such as, for example, "You become 'depressed' when you think that you 'always fail in document creation', don't you? Why did you feel 'anxiety' at that time? Tell us thought at that time in bit more detail." In addition to the outputting of the message for reconsideration of an automatic thought, the automatic thought eliciting unit 52 can also output a message for reconsideration of a feeling.

The automatic thought eliciting unit 52 may generate and output a sympathetic message for a finally obtained automatic thought, as one of interactive functions. For example, when the sentence of "I always fail in document creation" is obtained as the expression text of an automatic thought, the automatic thought eliciting unit 52 may output the sentence of "The thought of'I always fail in document creation' causes you to languish, doesn't it?". When a feeling is specified at this time, the automatic thought eliciting unit 52 may output the sentence of "The thought of 'I always fail in document creation.' causes you to have a 'depressed' feeling, doesn't it?".

The automatic thought eliciting unit 52 may allow information (input sentence, summary of automatic thought, result of classification of automatic thought, estimated feeling, worry term, evaluation value of worry term, presence or absence of vague expression for automatic thought, or the like) obtained in the present step processing to be stored in the information sharing unit 4 to enable the information to be utilized in the other steps.

### [Adaptive Thought Eliciting Step]

The adaptive thought eliciting step will be explained below. In the present exemplary embodiment, the adaptive thought eliciting unit 53 in the information eliciting unit 5 is associated with the step processing. The adaptive thought eliciting unit 53 executes various kinds of processing for eliciting "adaptive thought" from a client in the adaptive thought eliciting step. Specifically, the information elicited from the client by the adaptive thought eliciting unit 53 is information representing a current thought regarding a situation in which an automatic thought is generated. The adaptive thought eliciting unit 53 outputs, in the step, a message for prompting a user to input an adaptive thought for a situation in which the user's feeling is perturbed, and accepts an input of information (information representing adaptive thought) from the user.

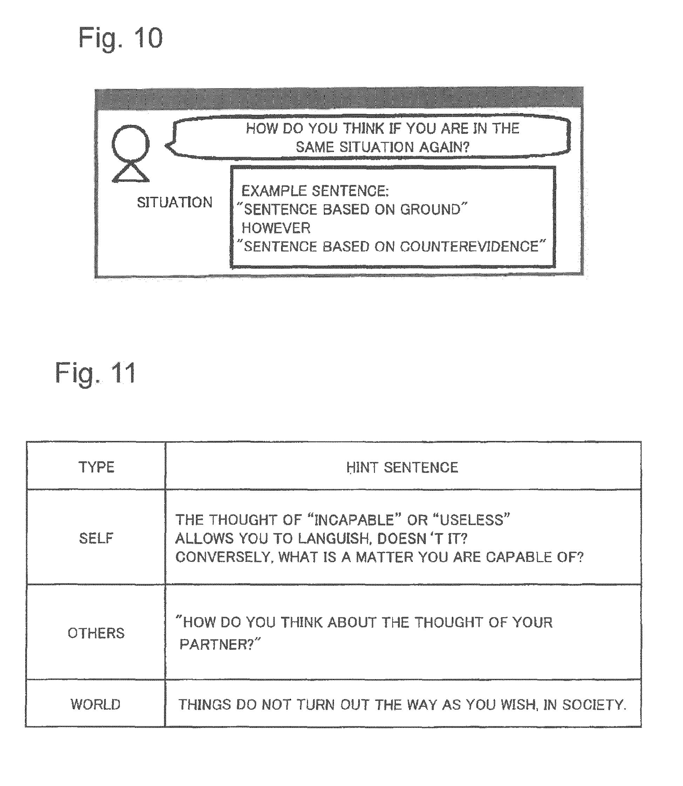

Fig. 9 and Fig. 10 are explanatory drawings illustrating examples of screens used in the adaptive thought eliciting step. The adaptive thought eliciting unit 53 allows a client to input a text representing "adaptive thought" thought by the client, for example, using such a screen as illustrated in Fig. 9 in the adaptive thought eliciting step. Such a screen includes a region for displaying a message for prompting entry of an adaptive thought, such as "How do you think if you are in the same situation again?", and an entry field for a situation.

If a ground and counterevidence are acquired in any of the previous steps, based on the ground and the counterevidence, the adaptive thought eliciting unit 53 may display, for example, as illustrated in Fig. 10, a sentence in which the ground and the counterevidence are joined to each other by an adversative conjunction, such as "'ground', 'however', 'counterevidence"', as an example sentence of an adaptive thought in an entry field. As a result, the adaptive thought eliciting unit 53 may allow the client to easily input an adaptive thought. When a plurality of grounds and a plurality of items of counterevidence are entered, the adaptive thought eliciting unit 53 may select the two grounds and two items that are most likely to be established as sentences, for the grounds and the items of counterevidence. When only a ground has been obtained, the adaptive thought eliciting unit 53 may enter a sentence of which the end is an adversative conjunction, such as "'ground' + however", in the entry field. This allows the client to easily write counterevidence leading to an adaptive thought.

Even when neither ground nor counterevidence has been obtained, the adaptive thought eliciting unit 53 may include the function of outputting a hint for counterevidence based on information obtained as an automatic thought.

In the adaptive thought eliciting step, the result of client's examination of a possible thought in the case of the same situation in future is allowed to be input. For the client to obtain an adaptive thought, it is important to pay attention to a fact different from the thought of the client, particularly to a fact, by which an automatic thought is denied, i.e., such as counterevidence. However, it is difficult for a person who concentrates his/her way of thinking on one direction, to find counterevidence by himself/herself. On the other hand, presenting of information to be an answer for the counterevidence is contrary to the concept of "eliciting from client himself/herself" in the cognitive behavior therapy. Thus, by presenting a hint enabling the client to perceive "fact different from his/her own thought", the adaptive thought eliciting unit 53 enables the client to perceive the counterevidence and to elicit the counterevidence as an adaptive thought.

The adaptive thought eliciting unit 53 may confirm whether or not a preconceived expression is included in, for example, a text document entered by a client in the entry field for an automatic thought, and generates a hint for counterevidence by selecting the hint based on the result of the confirmation. In that case, the adaptive thought eliciting unit 53 may prepare a sentence to be a hint beforehand. The adaptive thought eliciting unit 53 may specify a hint sentence that can be used among hint sentences prepared beforehand, for an input automatic thought. If a text document in which a ground is entered can be acquired in any of the previous steps, the adaptive thought eliciting unit 53 may also utilize the text document in which the ground is entered.

The hint sentence is specified, for example, by classifying automatic thoughts and grounds.

First, classification of automatic thoughts for specifying a hint sentence will be explained. It is considered that a negative automatic thought is prone to be directed at any one of "self", "others", and "world". By changing the way of the direction, the automatic thought can be interpreted from another viewpoint.

Automatic thoughts in which targets are classified into "world" are characterized by often including negative cognition for society and the public. Therefore, this system may registers, words representing, as expression examples, the unspecified number of targets, such as "society" and "public", usages of those words, and the like in association with information on the "world" category in a dictionary. The adaptive thought eliciting unit 53 may determine whether the target category of an automatic thought is "world" or not, by matching the expression of the automatic thought with the dictionary.

Automatic thoughts in which targets are classified into "others" are characterized by often including discontentment with specific persons. Therefore, this system may registers, words representing persons as expression examples, words representing specific persons, such as he and she, usages of those words, and the like in association with information representing the "others" category in a dictionary. The adaptive thought eliciting unit 53 may determine whether the target category of an automatic thought is "others" or not by matching the expression of the automatic thought with the dictionary. The adaptive thought eliciting unit 53 may also determine that an automatic thought is "others", when the expression is passive, because there are others.

The adaptive thought eliciting unit 53 may regard the target category of an automatic thought corresponding to neither "world" category nor "others" category as the "self" category.

The adaptive thought eliciting unit 53 may carry out selection of a hint or generation of an output message based on the type (target category) of such an automatic thought. The adaptive thought eliciting unit 53 may store, for example, hint sentences in association with the target categories of automatic thoughts as illustrated in Fig. 11. The adaptive thought eliciting unit 53 may select a hint sentence corresponding to the target category of an extracted automatic thought with reference to them.

Fig. 11 illustrates an example in which the hint sentence of "The thought of 'incapable' or 'useless' allows you to languish, doesn't it? Conversely, what is a matter you are capable of?" is registered, for example, in association with the "self" category. In addition, there is illustrated an example in which the hint sentence of "How do you think about the thought of your partner?" is registered in association with the "others" category. In addition, there is illustrated an example in which the hint sentence of "Things are unlikely to turn out as you wish in society." is registered in association with the "world" category.

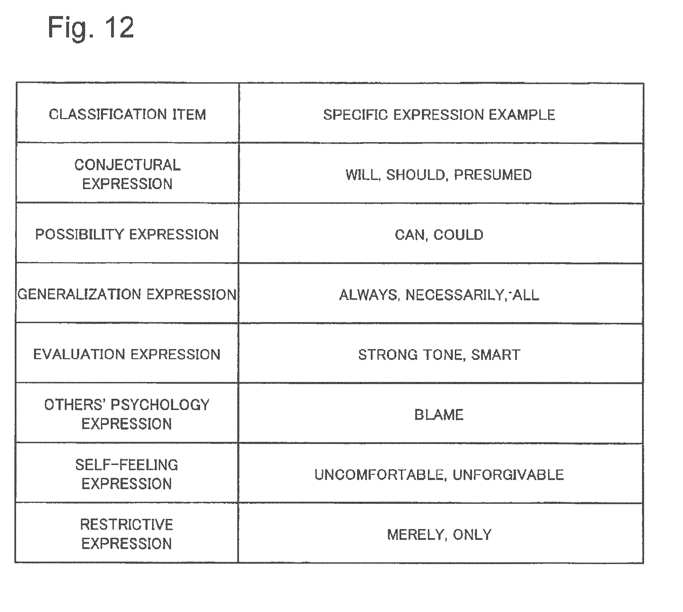

The adaptive thought eliciting unit 53 can also specify a hint sentence depending on classification of a preconceived expression included in an automatic thought. Preconceived expressions can be divided, depending on the expression contents thereof, into conjectural expression, possibility expression, generalization expression, evaluation expression, others' psychology expression, self-feeling expression, and restrictive expression. In this system, examples of specific expressions (words, usages thereof, and the like) are registered in a dictionary according to each classification as described above. Fig. 12 is an explanatory drawing illustrating an example of the dictionary in which expression examples are registered according to each classification item of the preconceived expressions.

The adaptive thought eliciting unit 53 may acquire a preconceived expression, for example, by a method similar to a method for extracting a worry term using the dictionary described above. In this case, the adaptive thought eliciting unit 53 regards, as extraction targets, phrases that are determined as "self" in the self/others determination, among phrases classified into the conjectural expression, the possibility expression, the generalization expression, the evaluation expression, and the self-feeling expression. In addition, the adaptive thought eliciting unit 53 regards, as extraction targets, phrases that are determined as "others", among phases that are classified into the others' psychology expression.

For example, it is assumed that the text of " 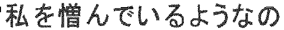 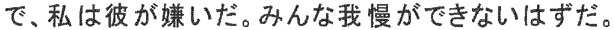 (Because he may hate me, I dislike him. Everybody should not be able to stand him.)" is input in an entry field for an automatic thought. In this case, as candidates for preconceived expressions, terms described below are extracted. That is, the term " 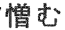 (a verb corresponding to "hate")" is extracted as the others' psychology expression, the term " 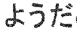 (an auxiliary verb corresponding "may")" is extracted as the conjectural expression, " 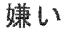 (a verb corresponding to "dislike")" is extracted as the self-feeling expression, " 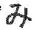 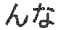 (a noun corresponding to "everybody")" is extracted as the generalization expression, " 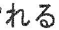 (an auxiliary verb corresponding to " not be able to ")" is extracted as the possibility expression, and " 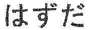 (an auxiliary verb corresponding to "should")" is extracted as conjectural expression.

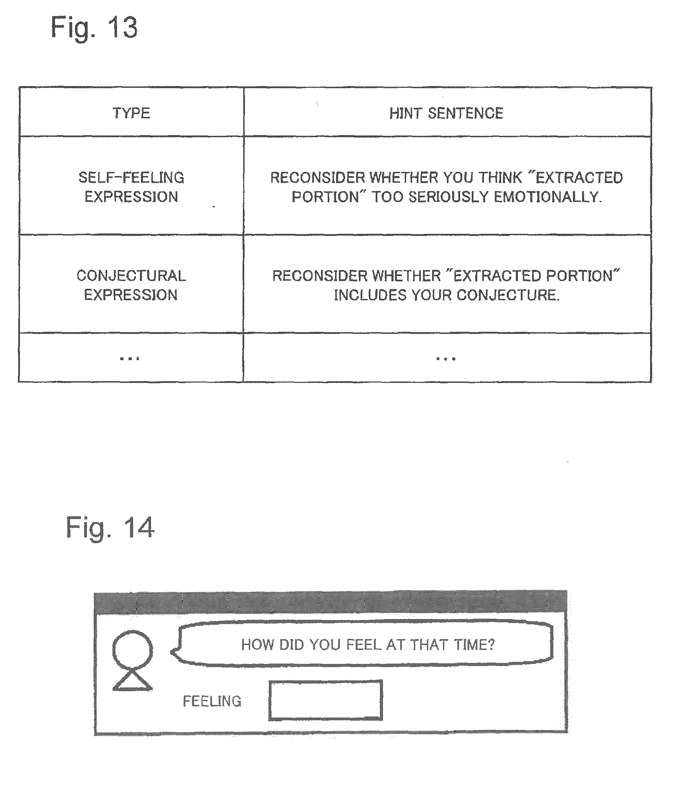

The adaptive thought eliciting unit 53 may also store hint sentences in association with the kinds of preconceived expressions, for example, as illustrated in Fig. 13, and selects a hint sentence corresponding to an extracted preconceived expression with reference to them.

Fig. 13 illustrates an example in which, for example, when there is a preconceived expression classified into the self-feeling expression, the hint sentence of "Reconsider whether you think 'extracted portion' too seriously emotionally." is registered. There is also illustrated an example in which when there is a preconceived expression classified into the conjectural expression, the hint sentence of "Reconsider whether 'extracted portion' includes your conjecture." is registered. As used herein, "extracted portion" included in each hint sentence means that an extracted phrase as a preconceived expression is applied to the "extracted portion". In such a manner, the adaptive thought eliciting unit 53 can also register the template of a hint sentence. The template may have content capable of outputting a message for thinking, based on the characteristic of the extracted expression, whether there is a fact pointing out the characteristic, or denying the characteristic.

For example, for the input sentence of "I always fail in document creation.", the adaptive thought eliciting unit 53 can extract the generalization expression of "always" using such registered data. Then, the adaptive thought eliciting unit 53 can generate the output message of "Is 'always' excessive generalization?" by applying the generalization expression of "always", to the registered templates of hint sentences.

When a ground/counterevidence eliciting step mentioned later is carried out before the adaptive thought eliciting step is executed, the adaptive thought eliciting unit 53 may also generate and output an example sentence for an adaptive thought using a ground or a ground and counterevidence, as illustrated in Fig. 10, as an interactive function.

Usually, since not only it is difficult for a worrying person to reconsider a current way of thinking but also it is difficult for the worrying person to know how to write properly, an adaptive thought is an item particularly difficult to enter. As a method for reconsidering the way of thinking, a method that organizes the thought of the person by dividing steps of the thought finely such as a ground and counterevidence, may be adopted, rather than a method that considers an adaptive thought from the beginning (see the ground/counterevidence step mentioned later).

The case of finely dividing steps of a thought in such a manner and eliciting a ground and counterevidence from a client, is assumed. In this case, as an interactive function, the adaptive thought eliciting unit 53 may create and output a template for an adaptive thought (example sentence) based on the ground and the counterevidence, for the purpose of allowing the client to understand an way of entering adaptive thought, in this step.

In this case, when one or more grounds and one or more items of counterevidence are obtained, the adaptive thought eliciting unit 53 select combinations in which junction of grounds and counterevidence is preferable among them, and generates templates based on them. Specifically, the adaptive thought eliciting unit 53 selects combinations of grounds and counterevidence that sentences that are constructed by using the groups are not devoid of meanings when the sentences are made as adaptive thoughts, from all the combinations of the grounds and the fire bells. The adaptive thought eliciting unit 53 may determine whether the sentences are devoid of meanings or not, for example, in such a manner as described below. First, the adaptive thought eliciting unit 53 collects sentence pairs that are joined to each other by adversative conjunctions. and sentence pairs that are not joined to each other by adversative conjunctions, from a network. The adaptive thought eliciting unit 53 uses the sentence pairs as positive examples and non-examples, to thereby construct a learning module capable of determining whether or not two arbitrary sentences are easily joined to each other by an adversative conjunction, when the two arbitrary sentences are input. The adaptive thought eliciting unit 53 inputs each combination of a ground and a counterstatement into the learning module, and presents, to a user, the combination determined to have the ground and the counterstatement that are most easily joined to each other by an adversative conjunction.

For example, it is assumed that the two sentences of "I was also pointed out about document creation before." and "I have had a bad memory since early times." as grounds for the automatic thought of "I always fail in document creation." In addition, it is assumed that the two sentences of "It is my first time to create this kind of a documentation." and " I have had experience that I was not pointed out about the document." are obtained as counterevidence. In this case, the adaptive thought eliciting unit 53 may select the group of "I was also pointed out about document creation before." and "I have had experience that I was not pointed out about the document.", which are most easily joined to each other by an adversative conjunction. Then, the adaptive thought eliciting unit 53 may create the template sentence for an adaptive thought, of " I was also pointed out about document creation before. However, I have had experience that I was not pointed out about the document."

The adaptive thought eliciting unit 53 may allow information (input sentence, type of automatic thought, presence or absence of preconceived expression, preconceived expression, result of classification of preconceived expression, and the like) obtained in step processing to be stored in the information sharing unit 4 to enable the information to be utilized in the other steps.

### [Mood Eliciting Step]

The mood eliciting step will be explained below. In present exemplary embodiment, the information eliciting unit 5 associated with the step processing is the mood eliciting unit 54. The mood eliciting unit 54 executes various kinds of processing for eliciting "mood" in the case of generation of an automatic thought from a client in the mood eliciting step. Specifically, the information elicited from the client by the mood eliciting unit 54 is information representing a mood(feeling) felt in the situation obtained in the situation eliciting step, particularly the most languishing feeling. In the step, the mood eliciting unit 54 outputs a message for prompting input of a feeling generated in a situation in which a user's feeling is perturbed and accepts the input of the information (information representing the feeling) from the user.

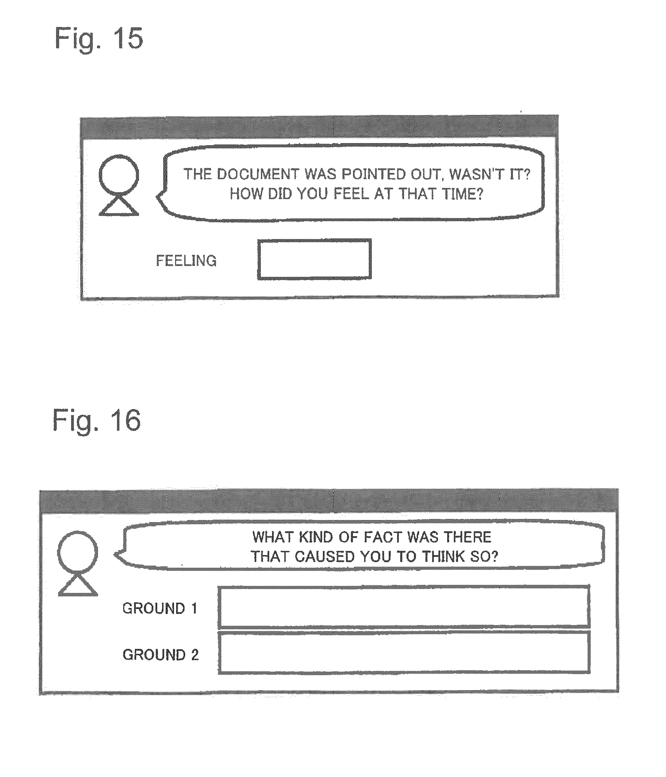

Fig. 14 and Fig. 15 are explanatory drawings illustrating examples of screens used in the mood eliciting step. In the mood eliciting step, the mood eliciting unit 54 allows a client to input a text representing "feeling" thought by the client, for example, using such a screen as illustrated in Fig. 14. Such a screen includes a region, in which a message for prompting entry of a feeling, such as "How did you feel at that time?", is output, and an entry field for a feeling.

The mood eliciting unit 54 may also output a sentence, in which the situation of a user is summarized, such as, for example, "The document was pointed out, wasn't it? How did you feel at that time?", as illustrated in Fig. 15, to thereby output a message for inquiring a feeling at the time of the situation. The mood eliciting unit 54 can allow the client to easily write the feeling that makes the client to languish, by clarifying that a feeling of what timing is inquired. A method for generating the message for summarizing a problematic situation may be similar to the method for generating a sympathetic message in the situation eliciting step. In the case of this processing, it is preferable to narrow down worry terms and feeling terms used in the message for summarizing a problematic situation to one. When the situation eliciting unit 51 stores a message applicable to the message for summarizing a problematic situation, generated in this processing, (i.e., sympathetic message for situation of user) into the information sharing unit 4, the mood eliciting unit 54 may read out and utilize the sympathetic message, stored in the information sharing unit 4.

The entry field for a feeling may have a free entry form (form in which a client can freely enter a phrase) or a selection input form (form in which some terms representing moods are prepared beforehand to allow a client to select the term from the terms). The entry field for a feeling may also have a combination of the selection input form and the free entry form.

As one of interactive functions, the mood eliciting unit 54 may also generate and output a sympathetic message for a feeling.

The mood eliciting unit 54 can specify a situation or an event allowing a client to languish and can output a sympathetic message for a feeling generated by the situation or the event, and thereby can build a therapeutic relationship (trust relationship), between the client and the system, that is important for mental health care. For building the trust relationship between the client and the system, for example, a relationship that can offer the client an expectation that the feeling of the client is precisely considered, and that a therapeutic effect may be obtained by utilizing the system, it is important to precisely specify a point, with which sympathy should be made, even if there are a plurality of terms corresponding to worries.

The mood eliciting unit 54 may uses, as an input, for example, information representing the feeling that is input by the client in the mood eliciting step, to generate and output a message sympathizing with the feeling. In the mood eliciting step, the mood eliciting unit 54 may allow the intensity of a feeling to be input with a numerical value or the like, and may include the value thereof as an input in the processing of generating the sympathetic message. The intensity of the feeling need not be a quantitative numerical value. The mood eliciting unit 54 may prepare selection items corresponding to the degrees of feelings, such as "very", "moderate", and "little", on a screen, to allow a client to enter or select a feeling as well as to select the degree of the feeling. The selection results may be included in the input in the processing of generating the sympathetic message.

In the processing of generating the sympathetic message, the mood eliciting unit 54 may carry out processing of extracting a negative word, for example, from a text representing a feeling. Then the mood eliciting unit 54 may change the content of the template sentence of a sympathetic message prepared beforehand, depending on the extracted word and the intensity of the feeling thereof.

The extraction of the negative word can be carried out by processing of matching with a dictionary. That is, words representing negative feelings and the represented feelings are pre-registered in the dictionary, and if a word registered in the dictionary is present in an input sentence, the mood eliciting unit 54 extracts the word. The negative words and sentence examples may be associated with each other in the dictionary so that natural messages are made. In this case, sentence examples with a plurality of patterns, like the case of expressing sympathy with a plurality of feelings and the case of expressing sympathy with a single feeling, may be prepared. As a result, the mood eliciting unit 54 can generate a more natural sympathetic message.

When intensity is applied to a feeling, the mood eliciting unit 54 may apply a modifier such as "strong", "considerable", or "little" depending on the magnitude of its value. When a plurality of extraction results are obtained and each of the results is converted into a representative feeling, if there are feelings overlapping with one another, the mood eliciting unit 54 may determine that those feelings overlapping with one another are strongly felt. The mood eliciting unit 54 may apply an importance degree to each word, and may regard the result of calculating the sum of those words in each representative feeling category, as the intensity of the feeling.

For example, it is assumed that the feeling terms of "sad", "anxiety", and "want to cry" are extracted from an input sentence. And also it is assumed that the input sentence is an input in which the intensity of "sad" is 60, the intensity of "anxiety" is 30, and the intensity of "want to cry" is 40.

In this case, first, the mood eliciting unit 54 may convert them into representative feelings, respectively. In this example, "sad" and "anxiety" are not converted because these are representative feelings, and "want to cry" is converted into "sad". As a result, extraction result in which the intensity of "sad" is 100 and the intensity of "anxiety" is 30, is obtained. In this case, the mood eliciting unit 54 may output the sympathetic message of "You were very sad and anxious, weren't you?" utilizing a template sentence prepared beforehand, such as "You were 'modifier with high intensity' 'feeling expression', 'modifier with low intensity' 'feeling expression', weren't you?". In this example, example sentences that include "sad" in the case of the former part of template sentence and "was sad" in the other cases, are registered with regard to the representative feeling of "sad". In addition, example sentences that include "anxious" in the case of the former part of template sentence and "anxiety" in the other cases, are registered with regard to the representative feeling of "anxiety". In addition, "very" is registered as a modifier with high intensity, and "(none)" is registered as a modifier with low intensity.

As described above, there can be expected the effect of building a therapeutic relationship (trust relationship) between a client and the system, that is important for mental health care, by outputting a sympathetic message for the situation or feeling of a user. The building of such a therapeutic relationship allows the client to honestly enter a feeling easily, and to use the system continuously. Such a therapeutic relationship leads to the effect of cognitive behavior therapy, specifically, improvement of a mood, and improvement of calming/changing of a feeling.

The mood eliciting unit 54 may also allow information (such as an input sentence, a feeling term, or a result of classification of a feeling) obtained in the step processing to be stored in the information sharing unit 4 and enable the information to be utilized in the other steps.

### [Ground/Counterevidence Eliciting Step]

The ground/counterevidence eliciting step will be explained below. In the present exemplary embodiment, the ground/counterevidence eliciting unit 55 in the information eliciting unit 5 is associated with the step processing. The ground/counterevidence eliciting unit 55 executes various kinds of processing for eliciting "ground" and "counterevidence" of an automatic thought in the ground/counterevidence eliciting step. Specifically, information elicited from a client by the ground/counterevidence eliciting unit 55 represents a ground why the client thinks such like the elicited automatic thought. In other words, the information elicited from the client by the ground/counterevidence eliciting unit 55 is information on the reason why the automatic thought is generated in the client, and another way of looking of the automatic thought, perceived by the client himself/herself. Such information is information, for example, on a fact that the client need not to think as elicited automatic thought, or on such a converse fact that may deny the elicited automatic thought. This step may be divided into two of a ground eliciting step and a counterevidence eliciting step. The ground/counterevidence eliciting unit 55 outputs a message for prompting input of the ground of an automatic thought and accepts an input of information (information representing the ground) from a user in the ground/counterevidence eliciting step. In addition, the ground/counterevidence eliciting unit 55 outputs a message for prompting input of counterevidence against an automatic thought and accepts an input of information (information representing the counterevidence) from the user.

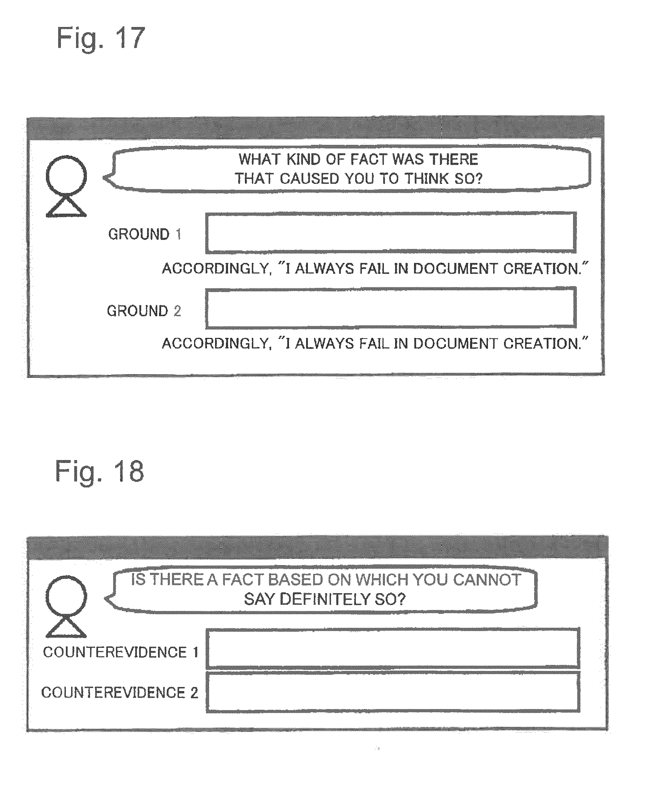

Fig. 16 and Fig. 17 are explanatory drawings illustrating examples of screens used in the ground eliciting step in the ground/counterevidence eliciting step. The ground/counterevidence eliciting unit 55 allows a client to input a text representing "ground" thought by the client, for example, using such an image as illustrated in Fig. 16 in the ground eliciting step. The screen illustrated in Fig. 16 includes a region for displaying a message for prompting entry of a ground of an automatic thought, such as "What kind of fact was there that caused you to think so?", and an entry field for a ground.

The ground/counterevidence eliciting unit 55 may also guide a client to be able to mention the ground of his/her own automatic thought, for example, using such a screen as illustrated in Fig. 17. Such a screen displays a message such as "Accordingly, 'automatic thought'" following the entry field for a ground. That is, following the entry field for a ground, the ground/counterevidence eliciting unit 55 may output a message in which a copulative conjunction such as "accordingly", "therefore", "so", or "hence" (conjunction functioning to lead the latter context as a result of the former context) and the summarization expression of an elicited automatic thought are joined to each other. The ground/counterevidence eliciting unit 55 may add a phrase such as "thought" into the end of a message, for example, like "Accordingly, 'summarization expression of elicited automatic thought' was thought.", to further clarify that an expression in quotation marks (") is an automatic thought.

In the example illustrated in Fig. 17, the ground/counterevidence eliciting unit 55 provides a plurality of entry fields for entering a plurality of grounds, and reduces the number of rows for writing in each entry field (for example, to one row). By this, the ground/counterevidence eliciting unit 55 performs the contrivance of allowing a user to organize content to be written and to write the content in brief expression.

In the ground eliciting step, the ground/counterevidence eliciting unit 55 also preferably check whether a preconceived speech (word) is included in an entered ground or not. This is because it is preferred that the ground of an automatic thought is based on a fact. However a person who concentrates his/her way of thinking on one direction often writes a preconceived thought as a ground. Thus, the ground/counterevidence eliciting unit 55 may extract a preconceived expression from a text input as a ground to determine whether or not the ground includes the preconceived thought of a client. When it is determined that the preconceived thought is included, the ground/counterevidence eliciting unit 55 may output a message for pointing out the determination result, or a preconceived expression, or a message for prompting description based on a fact.

The ground/counterevidence eliciting unit 55 can determine the presence or absence of a preconceived expression in an input text, by extracting the preconceived expression from the input text. A method for extracting the preconceived expression may be similar to the above-mentioned method for extracting a preconceived expression for an automatic thought. Specifically, the ground/counterevidence eliciting unit 55 uses a dictionary in which specific expression examples are registered depending on classification of preconceived expressions such as conjectural expression, possibility expression, generalization expression, evaluation expression, others' psychology expression, self-feeling expression, and restrictive expression. Then, the ground/counterevidence eliciting unit 55 may extract, as a preconceived expression, each matching expression obtained by comparing phrases obtained from the input text by executing morphological analysis and syntactic analysis and each expression example registered in the dictionary.

Then, the ground/counterevidence eliciting unit 55 may carry out self/others determination of the extracted expression. In this case, the ground/counterevidence eliciting unit 55 regards, as extraction targets, phrases that are determined as "self" in self/others determination, among phrases that are classified into the conjectural expression, the possibility expression, the generalization expression, the evaluation expression, and the self-feeling expression. In addition, the ground/counterevidence eliciting unit 55 regards, as extraction targets, phrases that determined as "others" in self/others determination, among phrases that are classified into the others' psychology expression.

Then the ground/counterevidence eliciting unit 55 may construct a message using a template registered in association with each kind, and a speech point (preconceived expression which is extracted). As an example of the template, for example, a message such as "Write in an expression that can be understood by other people, rather than a thinking pattern peculiar to you, like 'extracted portion'." is conceivable for the evaluation expression. In addition, as an example of the template, for example, a message such as "Write, specifically, what kind of fact based on which you thought so, without using an expression for presuming the feeling of a partner, like 'extracted portion'." is conceivable for the others' psychology expression. The content of the template may be content capable of outputting such a message explaining the characteristics of the extracted preconceived expression, and prompting writing in expression converse to the preconceived expression or writing in expression using a fact, rather than writing in such a preconceived expression.

For example, it is assumed that the texts of "I was also pointed out about document creation before." as a ground 1, and "My boss blamed me very much." as a ground 2 are input. In this case, the ground/counterevidence eliciting unit 55 may extract the preconceived expression (others' psychology expression) of "blamed" included in the text as the ground 2 using such registered data. Then, the ground/counterevidence eliciting unit 55 may output the pointing-out message of "Write, specifically, what kind of fact based on which you thought so, without using an expression for presuming the feeling of a partner, like 'blamed'." The ground/counterevidence eliciting unit 55 may also output the pointing-out message of "Write in an expression that can be understood by other people, rather than a thinking pattern peculiar to you, like 'very much'."

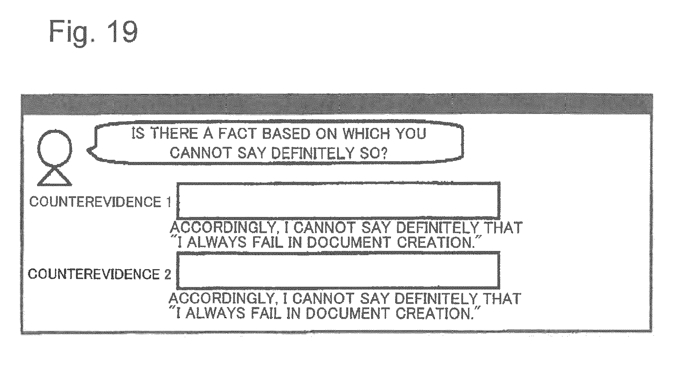

Fig. 18 and Fig. 19 are explanatory drawings illustrating examples of screens used in the counterevidence eliciting step in the ground/counterevidence eliciting step. The screen illustrated in Fig. 18 includes a region for displaying a message for prompting entry of counterevidence against an automatic thought, such as "Is there a fact based on which you cannot say definitely so?", and an entry field for counterevidence. The ground/counterevidence eliciting unit 55 allows a client to input a text representing "counterevidence" thought by the client, for example, using such an image as illustrated in Fig. 18 in the counterevidence eliciting step.

The ground/counterevidence eliciting unit 55 may also guide a client to mention counterevidence against his/her own automatic thought, for example, using the screen displaying the message of "Accordingly, I cannot say definitely that 'automatic thought'.", as illustrated in Fig. 19, following the entry field for counterevidence. In other words, the ground/counterevidence eliciting unit 55 may output such a message as described below in the form of following the entry field for counterevidence. That is, such a message may be a message in which a copulative conjunction such as "accordingly", "therefore", "so", or "hence" (conjunction functioning to lead the latter context as a result of the former context), the summarization expression of an elicited automatic thought, and an expression representing negation of necessity, such as "cannot say definitely" or "not necessarily" or expression representing "what should be" by a negative form, are joined.

The ground/counterevidence eliciting unit 55 may also present a hint for the above-mentioned counterevidence in the counterevidence eliciting step. In this case, the information of the ground acquired in the ground eliciting step can be used as an input. When the hint for the counterevidence is presented in the step, the presentation of the hint for counterevidence in the adaptive thought eliciting step may be omitted.

The ground/counterevidence eliciting unit 55 may allow information (such as input sentence, presence or absence of preconceived expression for ground, preconceived expression, or result of classification of preconceived expression) obtained in the step processing to be stored in the information sharing unit 4 and enable the information to be utilized in the other steps.

In the present exemplary embodiment, the flow control unit 3 and the information eliciting unit 5 are realized by an information processing apparatus operated according to a program, such as, for example, a CPU (Central Processing Unit). In addition, the information sharing unit 4 is realized by a storage apparatus, such as a memory or an HDD (Hard disk drive), or a database system. The mental health care system is realized by implementing these respective function units and storage units in a server apparatus connected to a network, or in a user terminal, such as a personal computer or a portable terminal, personally used by a user.

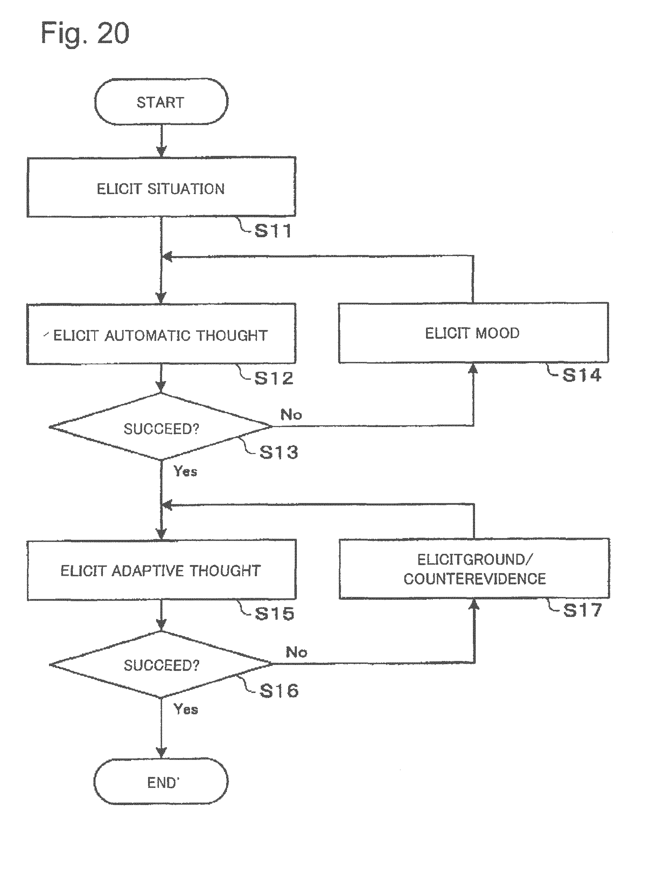

The operation of the present exemplary embodiment will be explained below. Fig. 20 is a flowchart illustrating an example of the operation of the mental health care system of the present exemplary embodiment. In the example illustrated in FIG. 20, first, the situation eliciting unit 51 carries out processing for eliciting "situation" from a client (step S11: situation eliciting step). For example, when user access to the top page of the system is detected, the flow control unit 3 informed of the access directs the situation eliciting unit 51 to start the processing, to thereby carry out this step.

The situation eliciting unit 51 displays, for example, a situation eliciting screen prepared beforehand (for example, the screen illustrated in Fig. 3) on a display included in the system or the display of a user terminal via the UI unit 1, when being directed to start the processing by the flow control unit 3. In addition, the situation eliciting unit 51 allows a user (client) to input a text representing "situation" thought by the user. The text representing the situation may be divided into a text representing "scene" or "time" and a text representing "event".

The situation eliciting unit 51 acquires information input into an entry field when detecting finishing of the entry of the text representing the situation by the user in the situation eliciting screen. Then, the situation eliciting unit 51 stores the information input into the entry field, as information representing "situation" acquired from the user, into the information sharing unit 4. In this case, the situation eliciting unit 51 may also generate a sympathetic message for the situation of the user and output the message on the same screen. The situation eliciting unit 51 may detect the finishing of the entry through an event notification by screen control from the UI unit 1.

In addition, the situation eliciting unit 51 detects that the user has input a manipulation for going to the next step, after entering the text representing the situation, in the situation eliciting screen. Then, the situation eliciting unit 51 notifies the flow control unit 3 of the completion of the situation eliciting step, in order to shift the processing to the next step. If there is acquired information other than the information entered in the entry field, the situation eliciting unit 51 stores the information acquired before this time into the information sharing unit 4. The situation eliciting unit 51 may detect the input of the manipulation for going to the next step through an event notification by screen control from the UI unit 1.

The flow control unit 3 proceeds to the automatic thought eliciting step when receiving the notification of the completion of the situation eliciting step. Specifically, the flow control unit 3 directs the automatic thought eliciting unit 52 to start the processing.

The automatic thought eliciting unit 52 carries out processing for eliciting "automatic thought" from the user (step S12: automatic thought eliciting step) following the previous step, when receiving the direction of the start of the processing from the flow control unit 3. The automatic thought eliciting unit 52 displays, for example, an automatic thought eliciting screen prepared beforehand (for example, the screen illustrated in Fig. 6) via the UI unit 1. And the automatic thought eliciting unit 52 allows the user to input a text representing "automatic thought" recognized by the user.

For example, if the situation acquired in step S11 of a feeling acquired in step S14 mentioned later are stored in the information sharing unit 4, the automatic thought eliciting unit 52 may output a display message including a feeling estimated from the situation acquired in step S11 or the feeling acquired in step S 14, in the automatic thought eliciting screen. The automatic thought eliciting unit 52 may also output a display message including the summarization expression of the situation acquired in step S11. The automatic thought eliciting unit 52 may also display the automatic thought eliciting screen divided into a screen for mentioning as many automatic thoughts as possible, and a screen for selecting the automatic thoughts. In this case, the automatic thought eliciting unit 52 may first display the screen for mentioning as many automatic thoughts as possible, and then display the screen for selecting one from the mentioned automatic thought.

In addition, the automatic thought eliciting unit 52 acquires information input into an entry field when detecting finishing of the entry of the text representing the automatic thought by the user in the automatic thought eliciting screen. In this case, the automatic thought eliciting unit 52 may call for attention if a vague expression is included. When the automatic thought and a mood contradict each other, the automatic thought eliciting unit 52 may also output a message for reconfirming an automatic thought or a feeling, or output a question message for delving into the automatic thought or the feeling by pointing out the contradiction. In addition, the automatic thought eliciting unit 52 stores the information on the automatic thought acquired in such a manner into the information sharing unit 4.

For a user who has difficulty in readily writing an automatic thought, a button for interactive support, or processing for checking the number of characters entered for predetermined time may also be included in the automatic thought eliciting screen. The automatic thought eliciting unit 52 may consider that failure occurs in the automatic thought eliciting step and may notify the flow control unit 3 of the failure, when detecting a press of the button, or detecting that the number of characters entered for the predetermined time is not more than a predetermined number. The automatic thought eliciting unit 52 may output a message for recommending switching of a flow without automatically changing a flow, and may notify the flow control unit 3 after obtaining the consent of the client.

The flow control unit 3 carries out control for proceeding to the mood eliciting step (No of step S13), when receiving the notification of the failure in the automatic thought eliciting step. Specifically, the flow control unit 3 directs the mood eliciting unit 54 to start the processing.

The mood eliciting unit 54 carries out processing for eliciting "mood" from the user (step S14: mood eliciting step) following the previous step, when receiving the direction of starting the processing from the flow control unit 3. The mood eliciting unit 54 displays, for example, a screen for eliciting a mood prepared beforehand (for example, the screen illustrated in Fig. 15) via the UI unit 1, and the mood eliciting unit 54 allows the user to input a text representing "mood" recognized by the user.

In this case, the mood eliciting unit 54 may indicate sympathy with the situation of the user by including the summarization expression of the situation acquired in step S11 in the display message in the mood eliciting screen.

The mood eliciting unit 54 acquires information input into an entry field and stores the information into the information sharing unit 4, when detecting finishing of the entry of the text representing the mood by the user in the mood eliciting screen. In this case, the mood eliciting unit 54 may also generate a sympathetic message for the feeling and output the message on the same screen.

When, the mood eliciting unit 54 detects the user entering the text representing the mood, followed by inputting a manipulation for going to the next step, in the mood eliciting screen, the mood eliciting unit 54 notifies the flow control unit 3 of the completion of the mood eliciting step in order to shift the processing to the next step. If there is acquired information other than the information entered in the entry field, the mood eliciting unit 54 stores the information acquired before this time into the information sharing unit 4.

The flow control unit 3 proceeds to the automatic thought eliciting step again (return to step S12) when receiving the notification of the completion of the mood eliciting step. Specifically, the flow control unit 3 may direct the automatic thought eliciting unit 52 to start the processing.

Meanwhile, in step S12, when the automatic thought eliciting unit 52 detects the user entering the text representing the automatic thought, followed by inputting a manipulation for going to the next step, in the automatic thought eliciting screen, then, the automatic thought eliciting unit 52 notifies the flow control unit 3 of the completion of the automatic thought eliciting step, in order to shift the processing to the next step. If there is acquired information other than the information entered in the entry field, the automatic thought eliciting unit 52 stores the information acquired before this time into the information sharing unit 4.

The flow control unit 3 proceeds the adaptive thought eliciting step (Yes of step S13, step S15) when receiving the notification of the completion of the automatic thought eliciting step. Specifically, the flow control unit 3 directs the adaptive thought eliciting unit 53 to start the processing.

The adaptive thought eliciting unit 53 carries out processing for eliciting "adaptive thought" from the user (step S 15: adaptive thought eliciting step) following the previous step when receiving the direction of the start of the processing from the flow control unit 3. The adaptive thought eliciting unit 53 displays, for example, an adaptive thought eliciting screen prepared beforehand (for example, the screen illustrated in Fig. 9 or Fig. 10) via the UI unit 1. And the adaptive thought eliciting unit 53 allows the user to input a text representing "adaptive thought" thought by the user.

For example, when a ground or counterevidence acquired in step S17 mentioned later is stored in the information sharing unit 4, the adaptive thought eliciting unit 53 may generate an example sentence of an adaptive thought based on the information and may display the example sentence in an entry field on the adaptive thought eliciting screen beforehand.

The adaptive thought eliciting unit 53 may verify whether or not the automatic thought includes a preconceived expression based on the information representing the automatic thought elicited in step S12. And, the adaptive thought eliciting unit 53 may display a hint which is obtained as a result of the verification, for counterevidence on the adaptive thought eliciting screen. The adaptive thought eliciting unit 53 may display the hint for counterevidence on the screen when the step is started, or may display the hint when detecting a press of a button for requesting the hint.

Then, the adaptive thought eliciting unit 53 acquires information input into an entry field when detecting finishing of the entry of the text representing the adaptive thought by the user. In this case, the adaptive thought eliciting unit 53 may output a message for calling for attention if the information input into the entry field (particularly, a counterevidence portion) includes a preconceived expression. The adaptive thought eliciting unit 53 stores the information finally acquired in such a manner as information representing the adaptive thought, into the information sharing unit 4.

In addition to the presentation of the hint for counterevidence, for a user who has difficulty in readily writing an adaptive thought, processing for a button for interactive support or processing for checking the number of characters entered for predetermined time may also be included in adaptive thought eliciting unit 53. The adaptive thought eliciting unit 53 may consider that failure occurs in the adaptive thought eliciting step when detecting detecting a press of the button for interactive support, or detecting the number of characters entered for the predetermined time being not more than a predetermined number. In this case, the adaptive thought eliciting unit 53 may notify the flow control unit 3 of the failure. The adaptive thought eliciting unit 53 may output a message for recommending switching of a flow without automatically changing a flow, and may notify the flow control unit 3 after obtaining the consent of the client.

The flow control unit 3 carries out control for proceeding the ground/counterevidence eliciting step (No of step S16) when receiving the notification of the failure in the adaptive thought eliciting step. Specifically, the flow control unit 3 directs the ground/counterevidence eliciting unit 55 to start the processing.

The ground/counterevidence eliciting unit 55 carries out processing for eliciting "ground" and "counterevidence" from the user (step S 17: ground/counterevidence eliciting step) following the previous step when receiving the direction of starting the processing from the flow control unit 3. The ground/counterevidence eliciting unit 55 displays, for example, a screen for eliciting a ground prepared beforehand (for example, the screen illustrated in Fig. 17) via the UI unit 1. Then, the ground/counterevidence eliciting unit 55 first allows the user to input a text representing "ground" thought by the user.

In this case, the ground/counterevidence eliciting unit 55 may guide the user to write easily the sentence which represents the reason of the automatic thought, by displaying the summarization expression of the automatic thought acquired in step S12, joined to a copulative conjunction, following an entry field on the ground eliciting screen.

The ground/counterevidence eliciting unit 55 acquires information input into the entry field when detecting finishing of the entry of the text representing the ground by the user. In this case, the ground/counterevidence eliciting unit 55 may output a message for calling attention if the information input into the entry field includes a preconceived expression. The ground/counterevidence eliciting unit 55 stores the information finally acquired in such a manner as information representing the ground, into the information sharing unit 4.

Then, the ground/counterevidence eliciting unit 55 carries out processing for eliciting "counterevidence" from the user. The ground/counterevidence eliciting unit 55 displays, for example, a counterevidence eliciting screen prepared beforehand (for example, the screen illustrated in Fig. 19) via the UI unit 1. And, the ground/counterevidence eliciting unit 55 allows the user to input a text representing "counterevidence" thought by the user.

The ground/counterevidence eliciting unit 55 may display a copulative conjunction, the summarization expression of the automatic thought acquired in step S12, and an expression representing negation of necessity or an expression representing "what should be" by a negative form, joined to each other, following an entry field on the counterevidence eliciting screen, and thereby, the ground/counterevidence eliciting unit 55 may guide counterevidence against the automatic thought to be easily written.

The ground/counterevidence eliciting unit 55 may verify whether or not the automatic thought includes a preconceived expression based on the information representing the automatic thought elicited in step S12, and may display an obtained hint as the result of verification for counterevidence on the counterevidence eliciting screen. The ground/counterevidence eliciting unit 55 may display the hint for counterevidence on the screen when the step is started or may display the hint when detecting a press of a button for a hint.

The ground/counterevidence eliciting unit 55 acquires information input into an entry field when detecting finishing of the entry of the text representing the counterevidence by the user, and stores the information to as information representing the counterevidence, into the information sharing unit 4. When the information representing the ground and the information representing the counterevidence are acquired in such a manner, the ground/counterevidence eliciting unit 55 notifies the flow control unit 3 of the completion of the ground/counterevidence eliciting step.

The flow control unit 3 proceed to the adaptive thought eliciting step again (return to step S 15) when receiving the notification of the completion of the ground/counterevidence eliciting step. Specifically, the flow control unit 3 may direct the adaptive thought eliciting unit 53 to start the processing.

Meanwhile, in step S15, when the adaptive thought eliciting unit 53 detects the user entering the text representing the adaptive thought, followed by inputting a manipulation for going to the next step, in the adaptive thought eliciting screen, then the adaptive thought eliciting unit 53 notifies the flow control unit 3 of the completion of the adaptive thought eliciting step, in order to shift the processing to the next step. If there is acquired information other than the information entered in the entry field, the adaptive thought eliciting unit 53 stores the information acquired before this time into the information sharing unit 4.

The flow control unit 3 finishes the processing of the mental health care based on cognitive reframing when receiving the notification of the completion of the adaptive thought eliciting step. The flow control unit 3 may then shift to a step for carrying out summarization without finishing the processing of the mental health care.

In the summarization step, the flow control unit 3 may display a message such as, for example, "Did your mood change?" to allow the user to input, e.g., the improvement degree of a current mood or feeling. And thereby, the flow control unit 3 may enable a confirmation of the effect, specifically, a confirmation of how much the mood is improved, or a confirmation of to what extent the feeling is calmed or changed. The flow control unit 3 may also allow, for example, the user whose mood is lightened to input a point leading to improvement. And, the flow control unit 3 may also allow the user to input a future problem. By allowing the user to input them, the user can recognize the point leading to the improvement and the problem, and the user can reconsider the feeling even by himself/herself in his/her subsequent daily life. When the mood is not lightened, it is effective for the user to recognize a fact in his/her subsequent daily life, thus, the flow control unit 3 may output a message for teaching a hint for the recognizing the fact. For example, the flow control unit 3 may give a hint for counterevidence based on the tendency of the preconception of an automatic thought, and display the message of "Find, in future daily life, whether there is such a fact."

In the mental health care system of the present exemplary embodiment, user evaluations such as the improvement degree of a feeling and an improvement point, can be stored together with the series of input contents (various kinds of the information obtained in each step) in one case, and can be thereby utilized in the next access. Each unit in the information eliciting unit 5 may specify a term that affects the user based on, for example, a message output when a variation in mood, (i.e., the effect of improving a feeling is large), an improvement point, or the like, and may preferentially select the term in the next access of the user. Each unit in the information eliciting unit 5 can also reflect the content input in the previous access to the interactive message in the next access. For example, the situation eliciting unit 51 may display a question such as "How about your problem?" at the time of the next start. For example, each unit in the information eliciting unit 5 may also output a message for making the user to aware the tendency of the automatic thought or feeling of the user, such as "You also had such a feeling due to such an occurrence before, didn't you?", during the step processing.

In addition, Fig. 20 illustrates an operation example in which the mood eliciting step for eliciting an automatic thought is carried out, when the result of the determination of the success or failure of eliciting of an automatic thought is determined as the failure. In addition, Fig. 20 illustrates an operation example in which the ground/counterevidence step for eliciting an adaptive thought is carried out when the result of the determination of the success or failure of eliciting of an adaptive thought is determined as the failure. However, these determinations of the success or failure may be omitted, and the operation of the system in the present exemplary embodiment may have a three-step structure of the situation eliciting step, the automatic thought eliciting step, and the adaptive thought eliciting step. In this case, user support is carried out by the hint presentation in the automatic thought eliciting step or the hint presentation in the adaptive thought eliciting step.

The operation of the system in the present exemplary embodiment can also have a five-step structure including the mood eliciting step and the ground/counterevidence step from the first, without the determination of success or failure.

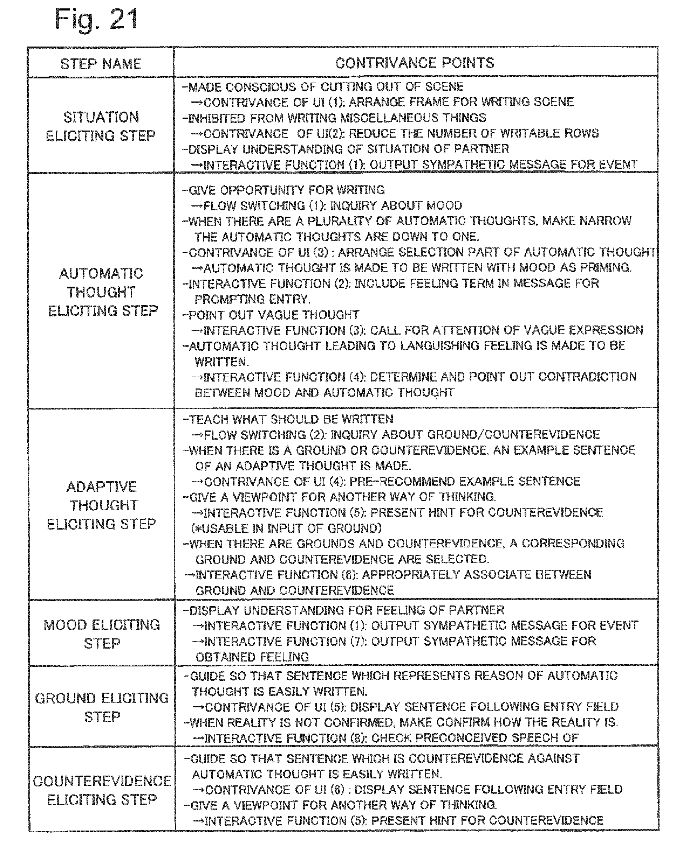

Fig. 21 is an explanatory drawing collectively illustrating representatives of the contrivance of UI and the contrivance of interactive functions according to each step as mentioned above. In addition to above mentioned contrivance, however, other various contrivances can be mentioned. For example, in the automatic thought eliciting step, the automatic thought eliciting unit 52 may check the habit of the way of thinking and output a pointing-out message or a hint for counterevidence such as, for example, "Thinking like black-and-white thinking appears, doesn't it?" or "Thinking like 'should thinking' appears, doesn't it?" depending on the pattern of the habit. Automatic thoughts can be divided into several patterns. Thus, the automatic thought eliciting unit 52 may check the pattern, to which the acquired automatic thought belongs, by using pattern recognition, and output a message corresponding to the pattern. Since understanding of the habit of the way of thinking facilitates advice, the automatic thought eliciting unit 52 may display a question for understanding the habit of a user in interaction. Alternatively, the automatic thought eliciting unit 52 may separately include a screen for providing a question in a questionnaire.

For a user who has used the system once, the information eliciting unit 5 may also display a message for understanding the time of using this system, for example, on a start screen or an end screen. In order to identify a user, the system may include a user authentication system, and may store, preferably in cooperation with the user authentication system, login information and previous entered contents according to each user in the authentication system. In such a case, the information eliciting unit 5 may display a message for understanding the time of using this system, based on the login information, or previously entered contents of a user, which is able to be referred together with the identifier of the user who logins, after the login. When this system is implemented in a user terminal, the information eliciting unit 5 may unconditionally regard users as the same user without processing of identifying a user, and may display a message for understanding the time of using this system based on a past login history or entered contents. The information eliciting unit 5 may refer to a final use day based on, for example, the history of using this system by a user, and may regard time after a lapse of a pre-determined period as the time of using this system, based on the final use day. The information eliciting unit 5 may also calculate an optimal elapsed period based on the frequency of use by the user, and regard the calculated period as the time of using this system. In the case of, e.g., using this system in a company or the like, this system may determine whether there is the tendency of depression or not, based on attendance information in cooperation with an attendance management system. Then, a user who had better be considered to receive mental health care service with the system may be notified of a guide for the system. This system may also notify, for example, a user with not less than the predetermined number of times of lateness and absence of a guide. This system may also determine, e.g., whether there is the tendency of depression or not based on the content of an email, for example, in cooperation with an email function. In addition, a user can also be notified of a guide for the system when it is determined that the user had better receive mental health care service with the system.

An input screen may also be resident on a terminal used by the user, and allow the user to start input willingly in the case of, e.g., the user wanting to complain.

The information eliciting unit 5 may also give a feeling, in which a user is remembered, for example, by outputting information unique to the user, such as "I see you after an interval of ... , don't I?", at the time of start. Thereby, this system can relax the mind of the partner to easily start mental health care service. The information eliciting unit 5 may output a message such as "You've worked hard in the meeting." as the information unique to the user after a meeting, for example, when cooperating with a scheduling management system. The information eliciting unit 5 may also estimate a state (for example, apparent age) utilizing a face image. In this case, the information eliciting unit 5 may output "Isn't your condition bad today?" when determining that the user looks older than usual. The information eliciting unit 5 may output "Your condition looks good today, doesn't it?" when determining that the user is younger than usual.

The information eliciting unit 5 may also output such a dialog as to be an ice break, such as "It is hot today, isn't it?", to easily start mental health care service, by using Web service outputting information such as weather.

In addition, the information eliciting unit 5 may also specify a native place based on the registered information, or input contents of a user, and put a dialect in an output message. By putting the dialect, a nostalgic feeling can be given, and therefore, mental care can also be performed due to nostalgia. The information eliciting unit 5 may acquire local event information utilizing Web service and output a message such as "I hear there is '***' in '***' this time." to relax the mind of the partner.

As a contrivance that common to all the steps, for example, when the information eliciting unit 5 is able to extract a matter like a situation or event causing a user to feel languishing, an automatic thought, a feeling, or an adaptive thought from input information, the information eliciting unit 5 may utilize the extracted matter in a message in subsequent steps. The information eliciting unit 5 may also output, for example, such a message for requiring a user to perform confirmation as described below utilizing information that has been extracted. That is, such a message may be the message for requiring a user to perform confirmation, such as "You felt '***' due to '***', didn't you?" or "You felt '***' and thought that '***' due to '***', didn't you?". Such a manner is effective for reconsideration of thinking because of allowing the user to be able to objectively view a situation, and a feeling and a thought generated at that time.

For example, the information eliciting unit 5 can extract the important portion of the words of a user and repeat (parrot) the portion to thereby improve a trust relationship with the user, and, as a result, can build a better therapeutic relationship.

For example, the information eliciting unit 5 may also change the facial expression of a character or a message depending on content input by a user in order to share a worrying state or pleasure.

As a contrivance common to the screens, for example, buttons for going to the next may also be unified into one color (for example, green). In the system in accordance with the present exemplary embodiment, the messages are output in balloon shapes in order to produce interactive feelings. In addition, in the system in accordance with the present exemplary embodiment, content input by a user can be confirmed on a screen. In addition, the system in accordance with the present exemplary embodiment, characters in a balloon are relatively enlarged so that a dialog gets into eyes first. Such contrivance on the screen can allow a user to easily input information.

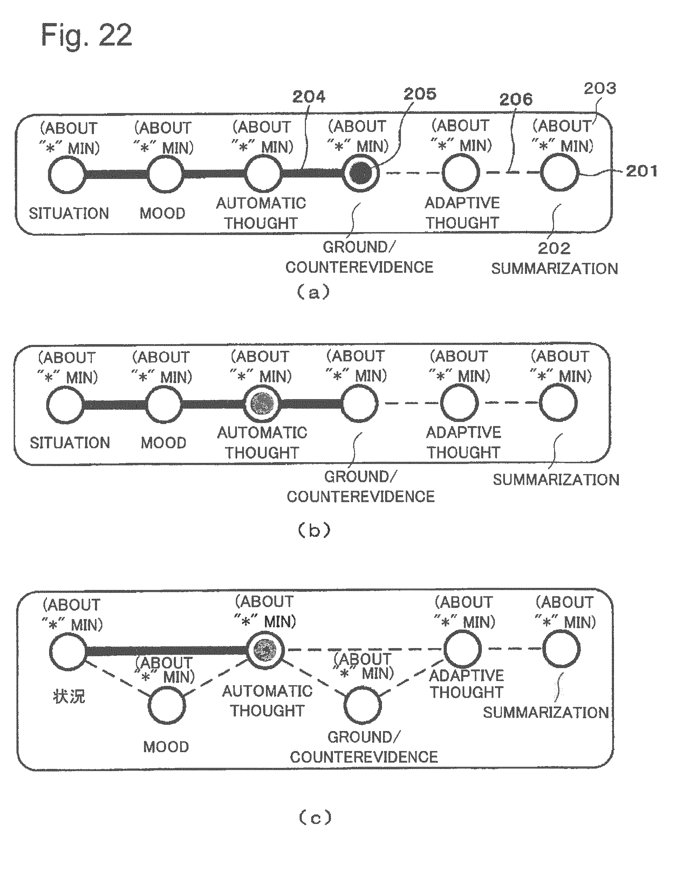

In the present exemplary embodiment, an overall flow, necessary time, and an indicator indicating past progress are always displayed on a screen as illustrated in Fig. 22, to allow a user, who is anxious about until when counseling continues, to recognize the end thereof. The indicator illustrated in Fig. 22 indicates the overall flow by joining and displaying white circles 201 corresponding to respective steps. Time information 203 applied to the upper portion of each white circle 201 indicates an estimation of time required for each step. A continuous line 204 by which white circles 201 are joined indicates that processing proceeds to steps joined by the line. A broken line 206 by which white circles 201 are joined indicates that steps joined by the line are unprocessed. A step with a white circle 201 on which a black circle 205 is drawn indicates a currently displaying step. By disposing the indicator with such usage, for example, in (a) of Fig. 22, it is understood that the processing proceeds up to a ground/counterevidence eliciting step and a screen corresponding to the ground/counterevidence eliciting step is currently displayed.

In addition, it is preferable that a user can freely take each entry form since the user himself/herself performs the operation of reconsidering his/her thought in cognitive reframing. Therefore, the system in accordance with the present exemplary embodiment is provided with the function of being able to return to a previous step at any time. Specifically, in the system in accordance with the present exemplary embodiment, clicking of the white circle 201 of an already entered step on the above-mentioned indicator causes the screen corresponding to the step to be displayed. For example, as illustrated in (b) of Fig. 22, there is assumed the case of wanting to reconsider the input of an automatic thought, in a state in which the processing proceeds up to the ground/counterevidence eliciting step. In this case, in the system in accordance with the present exemplary embodiment, a user may be allowed to return to the automatic thought eliciting step by, e.g., clicking the white circle 201 of the automatic thought eliciting step. In this case, the screen corresponding to the automatic thought eliciting step (i.e., screen for eliciting automatic thought) is displayed, and a text already input by the user once is displayed in the entry field. For acquiring the text input by the user, the system in accordance with the present exemplary embodiment may refer to information stored in the information sharing unit 4.

In (c) of Fig. 22, an example of the indicator in the case that the processing is branched into two or more, is illustrated. The function of displaying an overall flow and the function of returning to a previous step are not limited to such a method with an indicator.

In some cases, it is also effective to rely on a real counselor, it is preferable that past input information can be shared with the counselor on that occasion. Therefore, the interactive function unit 2 may list past input contents, and make the contents in a book (in printable display form) to enable the contents to be output. When the contents are made in the book, the interactive function unit 2 may enable classification and a sort based on a time series, a situation, a feeling, or the like. The output of the past input contents made in the list facilitates reconsideration by the user himself/herself.

The interactive function unit 2 may also include a looking-back mode, in which a past case example can be re-input, as an example of utilizing a history. In other words, the interactive function unit 2 may enable a case example which is concerned about in a history to be selected, and may allow user to enter the current way of thought considering the same situation. In such a looking-back mode, the interactive function unit 2 may start from the adaptive thought eliciting step or the ground/counterevidence eliciting step, for example, in the form of taking over a problem situation and an automatic thought entered in the past.

Although it is assumed that a text is input in all the examples described above, a voice may be input. In such a case, each unit in the information eliciting unit 5 may convert an input voice into a text, followed by carrying out the above-mentioned steps. An output form may also be a voice rather than a text. In such a case, each unit in the information eliciting unit 5 may carry out voice synthesis processing of an output message. Thereby, even a blind person is able to utilize this system.

As described above, for example, in accordance with the present exemplary embodiment, for eliciting information or a solution necessary for resolution from a client, contrivance of UI, support for entry, and an interactive function, such as a brief input, an output of a sympathetic/summarization message, and a response for compensating an undefined point are realized. Thereby, a client is able to enter information and a solution necessary for reconsidering a thought by the client himself/herself. As a result, effects by cognitive behavior therapy, specifically, improvement of a mood and improvement of calming/changing of a feeling can be expected.

In accordance with the present exemplary embodiment, for example, the system determines the degree of the difficulty of writing for the user based on elapsed time, the number of input characters, and the like, and if the writing is difficult for the user, the system controls to switch to the flow facilitating the writing. Therefore, operation time can be reduced for a practiced user while input support can be carefully performed for an unpracticed user.

### Exemplary Embodiment 2.

In the above explanation, the characteristics, configuration, and operation of the present invention are described by taking, as an example, the mental health care system for providing mental health care based on cognitive behavior therapy to a user with the tendency of depression. However, the present invention is applicable to systems used for correcting the cognitive distortions of users with the cognitive distortions, or for allowing the users to find the distortions, without limiting to the mental health care.

For example, the present invention can be applied to a system for supporting smoking cessation for a smoker. Specifically, the present invention can also be applied to a system for correcting a cognitive distortion to which a smoker is prone when the smoker attempts or is in the process of smoking cessation, or for allowing the smoker to find the distortion.

Because there are many persons who say "I am undone!" and give up smoking cessation when puffing even a cigarette during the smoking cessation, therefore, it may be effective to provide treatment focusing on a cognitive perspective.

For example, although an objective fact is just "One cigarette was puffed during smoking cessation.", a person who quits smoking may have the automatic thought of "I have a weak will. I fail in smoking cessation." In such a case, the system reconfigures cognition in the direction of preventing a jump to the conclusion of failing in smoking cessation even when puffing one cigarette. In other words, the system elicits advice or information, for the user to find a thought such as "If anything, you happily came out of it all with just one cigarette." or "Only puffing of one cigarette does not mean failure in smoking cessation." as an adaptive thought.

In a specific example, it is assumed that "I have a weak will. I fail in smoking cessation." is input as an automatic thought. In this case, the interactive function unit 2 may output a message representing sympathy, such as "You almost give up due to a sense of guilt, don't you?", for example, for words such as "have a weak will". The interactive function unit 2 may also present a hint such as "Do you really thus fail?", for example, for words such as "failure in smoking cessation". It can be expected that an adaptive thought such as "Indeed, only puffing of one cigarette does not mean failure. If anything, quitting smoking with just one cigarette means that the smoking cessation goes well." is elicited by presenting such a sympathetic message or a hint.

As described above, in uses other than mental health care, a system to which the present invention is applied can also create a message representing sympathy for an event or feeling occurring in a person concerned, for example, by carrying out various kinds of determination processing, such as self/others determination, of an extracted worry term or feeling term. In addition, for example, the system to which the present invention is applied can extract content pointing out a vague expression, a preconceived expression, or contradiction, included in information input by a user, and present a message for pointing it out or a message including a hint leading to an adaptive thought based on it.

The present invention can be applied to not only uses of supporting smoking cessation for smokers but also uses for reducing negative tendencies in life, e.g., for treating bulimia, anorexia, and lifestyle-related diseases. In each system, it is more preferable to register, in a dictionary, worry terms, feeling terms, and the like by considering the tendencies of subjects, depending on the use of the system.

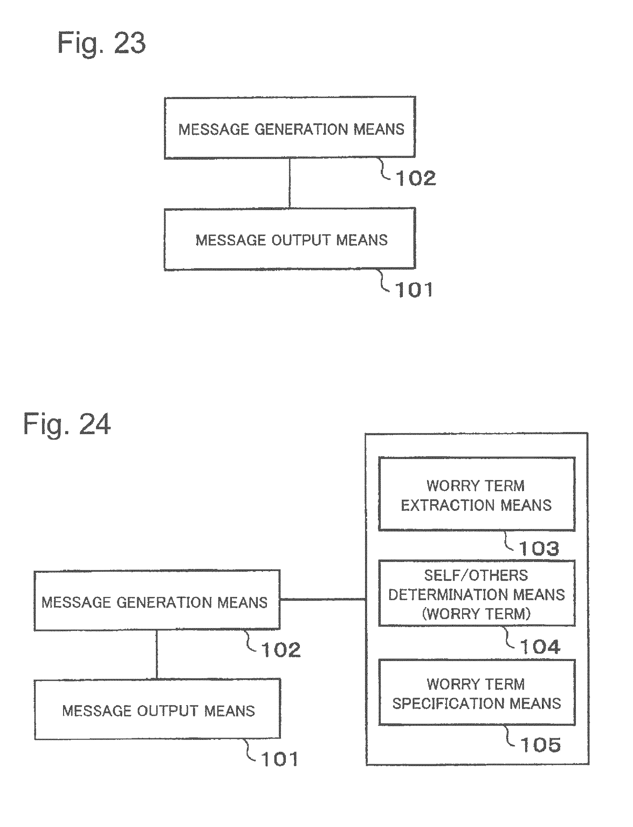

The outline of the present invention will be explained below. Fig. 23 is a block diagram illustrating the outline of a system for supporting correction of a cognitive distortion according to the present invention. As illustrated in Fig. 23, the system for supporting correction of a cognitive distortion according to the present invention includes: message output means 101 (for example, UI unit 1) that outputs an interactive message which is a message for eliciting necessary information from a user in each of steps; and message generation means 102 (for example, informational eliciting unit 5) that generates the interactive message in at least one step of the steps by utilizing information previously input by the user.

As used herein, each step refers to at least one of an automatic thought eliciting step including processing for outputting a message for prompting input of an automatic thought which is a thought generated in a situation in which a user's feeling is perturbed, and an adaptive thought eliciting step including processing for outputting a message for prompting input of an adaptive thought which is a thought that can be currently taken with regard to the situation.

The message generation means 102 may also generate, as the interactive message, a message including content representing sympathy for the situation or state (for example, state in which a certain feeling is generated) of the user using at least one of a worry term which is a phrase representing worry, and a feeling term which is a phrase representing a feeling, of the user, specified based on information input by the user.

The message generation means 102 may also generate, as the interactive message, a message including content for a hint or opportunity for allowing the user to input information on a purpose, generated based on information input by the user.

The message generation means 102 may also generate, as the interactive message, a message including content that points out a vague expression, a preconceived expression, or contradiction included in information input by the user.

As illustrated in Fig. 24, the system for supporting correction of a cognitive distortion according to the present invention may also further include worry term extraction means 103, self/others determination means 104, and worry term specification means 105. The worry term extraction means 103 extracts the worry term included in information input by the user and representing the situation, in which the user's feeling is perturbed, based on information on the worry term which is pre-stored. The worry term extraction means 103 corresponds to a module for extracting a worry term, included in, for example, the situation eliciting unit 51 or the adaptive thought eliciting unit 53. The self/others determination means 104 determines, using a predetermined determination method, whether or not the worry term extracted by the worry term extraction means 103 represents an event that is occurred in the user. The self/others determination means 104 corresponds to various determination modules included in, for example, the situation eliciting unit 51 and the adaptive thought eliciting unit 53. The worry term specification means 105 specifies a worry term included in the information, input by the user, on the situation, in which the user's feeling is perturbed, and representing the event that is occurred in the user, based on a determination result by the self/others determination means 104. The worry term specification means 105 corresponds to an integrated determination module included in, for example, the situation eliciting unit 51 or the adaptive thought eliciting unit 53. In such a case, the message generation means 102 may generate, as the interactive message, a message for repeating the summary of the event that is occurred in the user using at least one worry term specified by the worry term specification means 105. In such a case, the system may be a system for supporting correction of a cognitive distortion, further executing a situation eliciting step including processing for outputting a message for prompting input of information on the situation in which the user's feeling is perturbed.

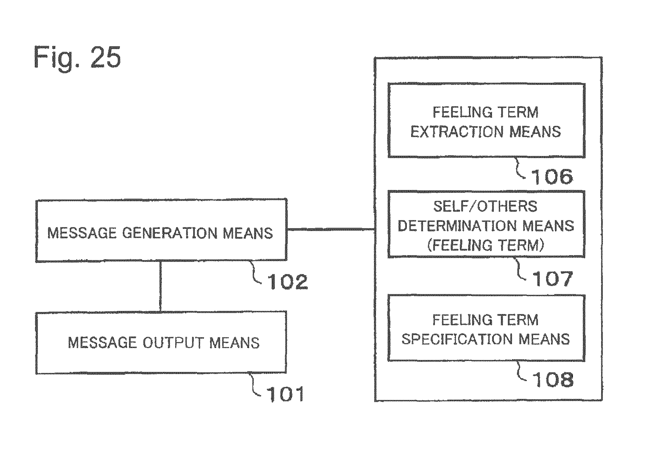

As illustrated in Fig. 25, the system for supporting correction of a cognitive distortion according to the present invention may also further include: feeling term extraction means 106 (module for extracting a feeling term, included in, for example, the situation eliciting unit 51 or the adaptive thought eliciting unit 53) that extracts the feeling term included in information input by the user and representing the situation, in which the user's feeling is perturbed, based on information on the feeling term which is pre-stored; self/others determination means 107 (various determination modules included in, for example, the situation eliciting unit 51 and the adaptive thought eliciting unit 53) that determines, using a predetermined determination method, whether or not the feeling term extracted by the feeling term extraction means 106 represents a feeling generated in the user; and feeling term specification means 108 (integrated determination module included in, for example, the situation eliciting unit 51 or the adaptive thought eliciting unit 53) that specifies a feeling term included in the information, input by the user, on the situation, in which the user's feeling is perturbed, and representing the feeling generated in the user, based on a determination result by the self/others determination means 107. In such a case, the message generation means 102 may generate, as the interactive message, a message for repeating the summary of the feeling generated in the user using at least one feeling term specified by the feeling term specification means 108. In such a case, the system may be a system for supporting correction of a cognitive distortion, further executing the situation eliciting step including the processing for outputting the message for prompting input of information on the situation in which the user's feeling is perturbed.

The self/others determination means 107 may carry out at least any one determination of, self/others determination for determining whether a subject in a sentence including an extracted worry term or feeling term is a self or not, negative determination for determining whether the worry term or the feeling term is negated or not, interrogative determination for determining whether the sentence including the worry term or the feeling term is an interrogative sentence or not, conjectural determination for determining whether the worry term or the feeling language is conjectural or not, and conditional determination for determining whether the worry term or the feeling language is a conditional term or not, to determine whether the worry term or the feeling term represents an event actually occurred in the user or a feeling generated in the user.

The self/others determination may also determine whether the subject in the sentence is a self or not based on the genitive of a speaker and on the voice of a verb in the sentence including the extracted worry term or feeling term.

The negative determination may also determine whether the extracted worry term or feeling term is negated or not, based on the number of negative expressions appearing behind the extracted worry term or feeling term.

The interrogative determination may also determine whether the worry term or the feeling term is included in an interrogative sentence or not based on whether or not an interrogative expression appears behind the extracted worry term or feeling term.

The conjectural determination may also determine whether the worry term or the feeling term is conjectural or not based on whether or not a conjectural expression appears behind the extracted worry term or feeling term.

The conditional determination may also determine whether the worry term or the feeling term is a conditional term or not based on whether or not a term which constitutes conditional clause is present in a character string behind the extracted worry term or feeling term.

The self/others determination means 107 may also use a machine learning module that learned, in advance, a result of person's determination, for a sentence including a phrase or expression that can be the worry term or the feeling term, whether or not the phrase or the expression is a matter actually occurred in the user, to determine whether or not the extracted worry term or feeling term represents an event actually occurred in the user or a feeling generated in the user.

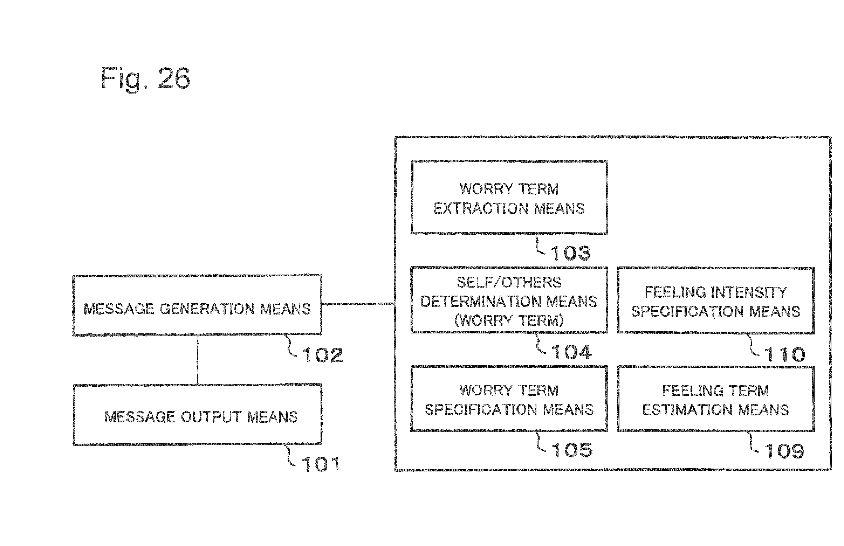

As illustrated in Fig. 26, the system for supporting correction of a cognitive distortion according to the present invention may also further include feeling estimation means 109 (feeling estimation module included in, for example, the situation eliciting unit 51) that estimates a feeling generated in the user from the worry term specified by the worry term specification means 105 based on pre-stored information on a correspondence relationship between the worry term and the feeling,. In such a case, the message generation means 102 may generate, as the interactive message, a message representing the user's feeling using at least one feeling term representing the feeling estimated by the feeling estimation means 109. Further, in combination with the configuration illustrated in Fig. 25, the message generation means 102 may generate, as the interactive message, a message representing the user's feeling using at least one feeling term representing the feeling estimated by the feeling term estimation means 109 when the feeling term specification means 108 is not able to specify the feeling term that is included in information, input by the user, on an event in which the user's feeling is perturbed, and represents the feeling generated in the user.

As illustrated in Fig. 26, the system for supporting correction of a cognitive distortion according to the present invention may further include feeling intensity specification means 110 (for example, a module for extracting a negative word, or a module for calculating intensity based on the multiplicity or importance of a word, included in the mood eliciting unit 54) that specifies a representative feeling and the intensity thereof based on information input by the user and representing the user's feeling. In such a case, based on the representative feeling specified by the feeling intensity specification means 110 and on the intensity thereof, the message generation means 102 may generate, as the interactive message, a message which represents sympathy to the user's feeling and of which the expression differs according to the intensity. In such a case, the system may be a system for supporting correction of a cognitive distortion, further executing a mood eliciting step including processing for outputting a message for prompting input of a feeling generated in the user in the situation in which the user's feeling is perturbed.

The message generation means 102 may also generate a message repeating or representing a feeling generated in the user as an interactive message displayed in an introduction of the message for prompting input of an automatic thought in the automatic thought eliciting step when generating the message repeating or representing the feeling generated in the user.

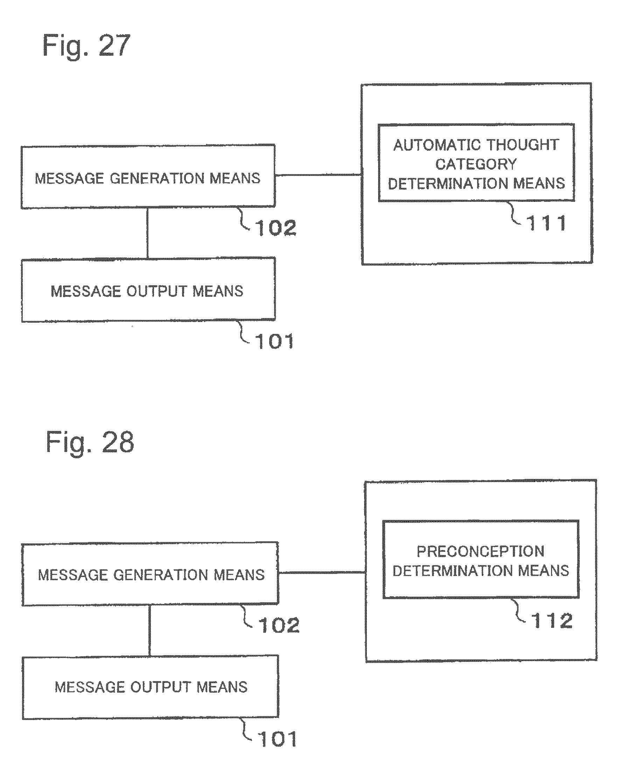

As illustrated in Fig. 27, the system for supporting correction of a cognitive distortion according to the present invention may further include automatic thought category determination means 111 (module for classifying the category of an automatic thought included in, for example, the automatic thought eliciting unit 52) that determines whether or not the automatic thought of the user corresponds to any one of predetermined categories based on information on words that are likely used in expressions of negative automatic thoughts, or on usages thereof, and that are pre-stored according to the predetermined categories into which automatic thoughts are classified, based on targets at which the negative automatic thoughts are prone to be directed. In such a case, the message generation means 102 may generate, as the interactive message, a message representing a hint for allowing the user to input an adaptive thought or counterevidence according to the corresponding category, prepared beforehand, when it is determined that the automatic thought, of the user, corresponds to any one of the predetermined categories as a result of the determination by the automatic thought category determination means 111.

As illustrated in Fig. 28, the system for supporting correction of a cognitive distortion according to the present invention may further include preconception determination means 112 (module for extracting a preconceived expression, included in, for example, the automatic thought eliciting unit 52) that determines whether a preconceived expression is present or not in an expression of an automatic thought of the user by extracting the preconceived expression included in information representing the automatic thought input by the user, based on information on words that are likely used in preconceived expressions or on usages thereof, pre-stored according to predetermined categories into which the preconceived expressions are classified according to expression contents thereof. In such a case, the message generation means 102 may generate a message representing a hint according to the predetermined category into which the preconceived expression is classified, for allowing the user to input an adaptive thought or counterevidence when the preconception determination means 112 determines that the preconceived expression is present.

In the configuration illustrated in Fig. 28, the message generation means 102 may generate, as the interactive message, a message for pointing out the preconceived expression when the preconception determination means 112 determines that the preconceived expression is present.

The message generation means 102 may generate, as the interactive message, a message representing a template for an expression of an adaptive thought as a hint for allowing the user to input an adaptive thought using a ground or the ground and counterevidence specified by information input by the user. In such a case, the system may be a system for supporting correction of a cognitive distortion, executing a ground/counterevidence eliciting step including processing for outputting a message for prompting input of a ground of an automatic thought and processing for outputting a message for prompting input of counterevidence against the automatic thought.

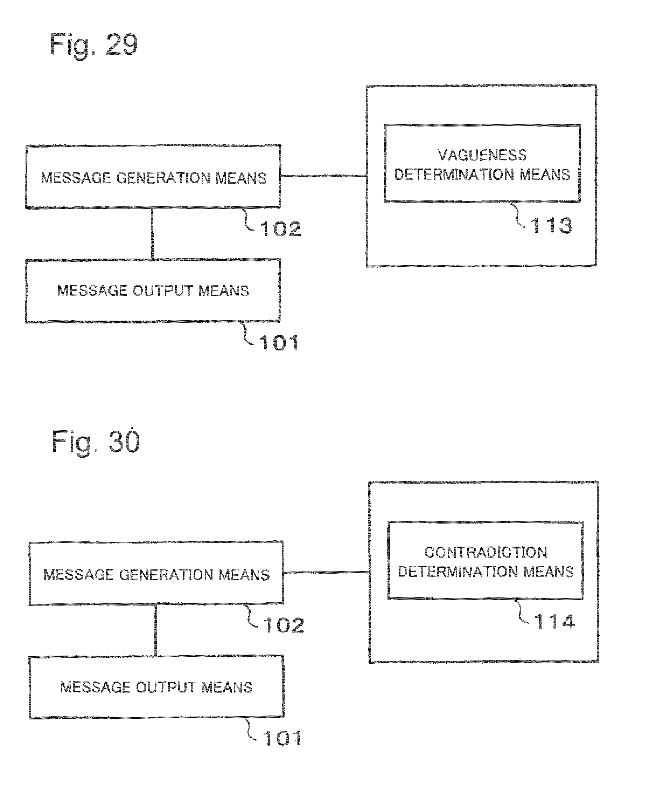

As illustrated in Fig. 29, the system for supporting correction of a cognitive distortion according to the present invention may further include vagueness determination means 113 (module for extracting a vague expression included in, for example, the automatic thought eliciting unit 52) that determines whether a vague expression is present or not in an expression of an automatic thought of the user by extracting the vague expression included in information representing the automatic thought input by the user, based on information on pre-stored vague expressions. In such a case, the message generation means 102 may generate, as the interactive message, a message for pointing out the vague expression when the vagueness determination means 113 determines that the vague expression is present.

In the configuration illustrated in Fig. 29, the message generation means 102 may further generate, as the interactive message, a message for proposing to correct vague expression to have an end in definite form with reference to a way of expression of an automatic thought when the vague expression determination means 113 determines that the vague expression is present.

As illustrated in Fig. 30, the system for supporting correction of a cognitive distortion according to the present invention may further include contradiction determination means 114 (module for determining contradiction included in, for example, the automatic thought eliciting unit 52) that determines the presence or absence of contradiction between an automatic thought and a feeling, based on an automatic thought category in which information representing the automatic thought of the user or the automatic thought of the user is classified into a predetermined category, and on a feeling category in which information representing the user's feeling or the user's feeling when the automatic thought is generated, is classified into a predetermined category. In such a case, the message generation means 102 may generate, as the interactive message, a message for pointing out contradiction when the contradiction determination means 114 determines that there is the contradiction between the automatic thought and the feeling.

In the configuration in Fig. 30, the message generation means 102 may further generate, as the interactive message, a message for reconfirming the automatic thought or the feeling, or for delving into the automatic thought or the feeling when the contradiction determination means 114 determines that the contradiction is present between the automatic thought and the feeling.

In the system for supporting correction of a cognitive distortion according to the present invention, two or more of the configurations illustrated in Fig. 23 to Fig. 30 may also be combined.

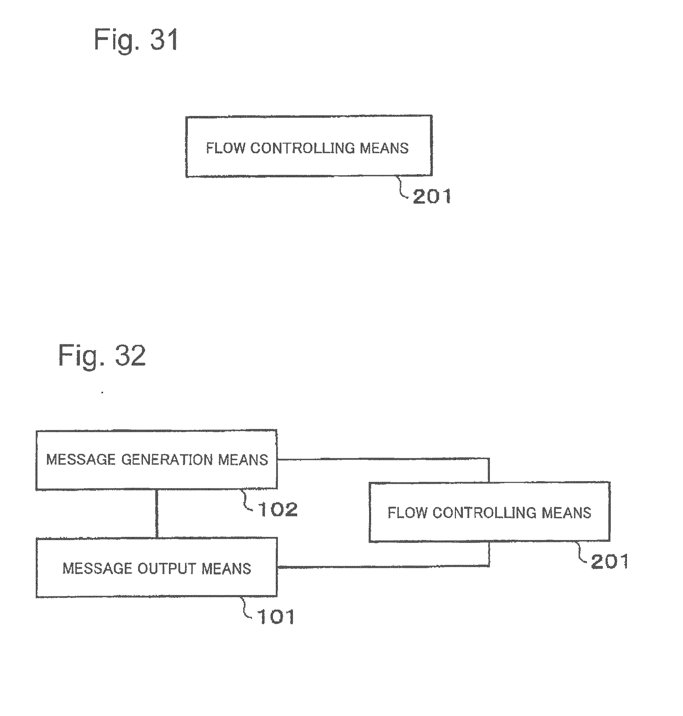

Fig. 31 is a block diagram illustrating another configuration example of a system for supporting correction of a cognitive distortion according to the present invention. As illustrated in Fig. 31, the system for supporting correction of a cognitive distortion according to the present invention includes flow controlling means 201 (for example, flow control unit 3) that controls a sequence of execution of each step. The flow controlling means 201 carries out at least an automatic thought eliciting step and an adaptive thought eliciting step in this sequence as a basic flow, and carries out flow control of, discontinuing the automatic thought eliciting step or the adaptive thought eliciting step, when determining that user input does not proceed in the automatic thought eliciting step or the adaptive thought eliciting step, and proceeding to a predetermined step including processing for outputting a message for prompting input of information for an opportunity or hint for input by a user in the discontinued step.

The flow controlling means 201 may also carry out flow control of proceeding to a mood eliciting step including processing for outputting a message for prompting input of a feeling generated in a user in a situation, in which a user's feeling is perturbed, when determining that user input does not proceed in the automatic thought eliciting step.

The flow controlling means 201 may also carry out flow control of proceeding to a ground/counterevidence eliciting step including processing for outputting a message for prompting input of a ground of an automatic thought, and processing for outputting a message for prompting input of counterevidence against the automatic thought, when determining that user input does not proceed in the adaptive thought eliciting step.

In the system for supporting correction of a cognitive distortion according to the present invention, as representatively illustrated in Fig. 32, the configuration including the message output means 101 and the message generation means 102 illustrated in Fig. 23 to Fig. 30 and the configuration including the flow controlling means 201 illustrated in Fig. 31 can also be combined.

A system for supporting correction of a cognitive distortion according to the present invention may include a screen for eliciting, from a user, information about a situation in which a user's feeling is perturbed, wherein the screen includes an entry field for writing a scene and an entry field for writing an event, which are separately disposed.

A system for supporting correction of a cognitive distortion according to the present invention may include a screen for eliciting, from a user, information about a situation that a user's feeling is perturbed, wherein the screen includes an entry field in which the user can make an entry and an apparent space available for the entry is limited to a size with not more than the predetermined number of characters.

A system for supporting correction of a cognitive distortion according to the present invention may include a screen for eliciting, from a user, an automatic thought which is a thought generated in the user in a situation in which a user's feeling is perturbed, wherein the screen includes a screen including a plurality of entry fields for allowing a user to enter an automatic thought, and a screen including a selection function for, when a plurality of automatic thoughts are entered, selecting one of the automatic thoughts.

A system for supporting correction of a cognitive distortion according to the present invention may include a screen for eliciting, from a user, an adaptive thought which is a thought that can be currently taken by the user with regard to a situation in which a user's feeling is perturbed, wherein an example sentence of the adaptive thought, which is generated from a ground which is a reason for generating an automatic thought which has been previously acquired from the user or the ground and counterevidence against the automatic thought, is displayed on the screen.

A system for supporting correction of a cognitive distortion according to the present invention may include a screen for eliciting, from a user, a ground of an automatic thought which is a thought generated in the user in a situation in which a user's feeling is perturbed, wherein a sentence in which a copulative conjunction and an summarization expression of an automatic thought are joined is displayed in form of following an entry field for a ground, on the screen.

The present invention has been explained above with reference to the exemplary embodiments and the examples. However, the present invention is not limited to the exemplary embodiments and examples described above. In the configurations and details of the present invention, various modifications that can be understood by those skilled in the art can be made within the scope of the present invention.

This application claims priority based on Japanese Patent Application No. 2012-255033, filed on November 21, 2012, the entire disclosure of which is incorporated herein.

### [Industrial Applicability]

The present invention is not limited to mental health care by cognitive reframing but is preferably applicable to uses of eliciting "situations", "thoughts", and "feelings" thought by users from the users as well as uses of checking the presence or absence of preconceived expressions and vague expressions from them in systems dealing with the consciousness of clients.

### [Reference signs List]

1
- UI: unit
- 2: Interactive function unit
- 3: Flow control unit
- 4: Information sharing unit
- 5: Information eliciting unit
- 51: Situation eliciting unit
- 52: Automatic thought eliciting unit
- 53: Adaptive thought eliciting unit
- 54: Mood eliciting unit
- 55: Ground/counterevidence eliciting unit
- 101: Message output means
- 102: Message generation means
- 103: Worry term extraction means
- 104: Self/others determination means (worry term)
- 105: Worry term specification means
- 106: Feeling term extraction means
- 107: Self/others determination means (feeling term)
- 108: Feeling term specification means
- 109: Feeling term estimation means
- 110: Feeling intensity specification means
- 111: Automatic thought category determination means
- 112: Preconception determination means
- 113: Vagueness determination means
- 114: Contradiction determination means
- 201: Flow controlling means

## Claims

1. A system, for supporting correction of a cognitive distortion, which is an interactive system that executes an automatic thought eliciting step comprising processing for outputting a message for prompting input of an automatic thought which is a thought generated in a user in a situation in which a user's feeling is perturbed, and an adaptive thought eliciting step comprising processing for outputting a message for prompting input of an adaptive thought which is a thought that can be currently taken by the user with regard to the situation,
wherein the system comprises:
message output means that outputs an interactive message which is a message for eliciting necessary information from the user in each of the steps; and
message generation means that generates the interactive message in at least one step of the steps by utilizing information previously input by the user.

2. The system for supporting correction of a cognitive distortion according to claim 1, wherein
the message generation means generates, as the interactive message, a message comprising content representing sympathy for the situation or state of the user using at least one of a worry term which is a phrase representing worry, and a feeling term which is a phrase representing a feeling, of the user, specified based on information input by the user.

3. The system for supporting correction of a cognitive distortion according to claim 1 or claim 2, wherein
the message generation means generates, as the interactive message, a message comprising content for a hint or opportunity for allowing the user to input information on a purpose, generated based on information input by the user.

4. The system for supporting correction of a cognitive distortion according to any one of claim 1 to claim 3, wherein
the message generation means generates, as the interactive message, a message comprising content that points out a vague expression, a preconceived expression, or contradiction included in information input by the user.

5. The system, for supporting correction of a cognitive distortion according to any one of claim 1 to claim 4, further executing a situation eliciting step comprising processing for outputting a message for prompting input of information on the situation in which the user's feeling is perturbed, the system further comprising:
worry term extraction means that extracts the worry term included in information input by the user and representing the situation, in which the user's feeling is perturbed, based on information on a pre-stored worry term;
self/others determination means that determines, using a predetermined determination method, whether or not the worry term extracted by the worry term extraction means represents an event that is occurred in the user; and
worry term specification means that specifies the worry term included in the information, input by the user, on the situation, in which the user's feeling is perturbed, and representing the event that is occurred in the user, based on a determination result by the self/others determination means,
wherein the message generation means generates, as the interactive message, a message for repeating the summary of the event that is occurred in the user using at least one worry term specified by the worry term specification means.

6. The system for supporting correction of a cognitive distortion according to any one of claim 1 to claim 5, executing the situation eliciting step comprising the processing for outputting the message for prompting input of information on the situation in which the user's feeling is perturbed, the system further comprising:
feeling term extraction means that extracts the feeling term included in information input by the user and representing the situation, in which the user's feeling is perturbed, based on information on a pre-stored feeling term;
self/others determination means that determines, using a predetermined determination method, whether or not the feeling term extracted by the feeling term extraction means represents a feeling generated in the user; and
feeling term specification means that specifies the feeling term included in the information, input by the user, on the situation, in which the user's feeling is perturbed, and representing the feeling generated in the user, based on a determination result by the self/others determination means,
wherein the message generation means generates, as the interactive message, a message for repeating the summary of the feeling generated in the user using at least one feeling term specified by the feeling term specification means.

7. The system for supporting correction of a cognitive distortion according to claim 5 or claim 6, further comprising:
worry term extraction means that extracts the worry term included in information input by the user and representing the situation, in which the user's feeling is perturbed, based on information on a pre-stored worry term;
self/others determination means that determines, using a predetermined determination method, whether or not the worry term extracted by the worry term extraction means represents an event that is occurred in the user;
worry term specification means that specifies a worry term included in the information, input by the user, on the situation, in which the user's feeling is perturbed, and representing the event that is occurred in the user, based on a determination result by the self/others determination means; and
feeling estimation means that estimates a feeling generated in the user from the worry term specified by the worry term specification means based on pre-stored information on a correspondence relationship between the worry term and the feeling,
wherein the message generation means generates, as the interactive message, a message representing the user's feeling using at least one feeling term representing the feeling estimated by the feeling estimation means.

8. The system for supporting correction of a cognitive distortion according to any one of claim 1 to claim 7, further executing a mood eliciting step comprising processing for outputting a message for prompting input of a feeling generated in the user in the situation in which the user's feeling is perturbed, the system further comprising:
feeling intensity specification means that specifies a representative feeling and the intensity thereof based on information input by the user and representing the user's feeling,
wherein based on the representative feeling specified by the feeling intensity specification means and on the intensity thereof, the message generation means generates, as the interactive message, a message which represents sympathy to the user's feeling and of which the expression differs according to the intensity.

9. The system for supporting correction of a cognitive distortion according to any one of claim 6 to claim 8, wherein
the message generation means generates a message repeating or representing a feeling generated in the user as an interactive message displayed in an introduction of the message for prompting input of an automatic thought in the automatic thought eliciting step.

10. The system for supporting correction of a cognitive distortion according to any one of claim 1 to claim 9, further comprising:
automatic thought category determination means that determines whether or not the automatic thought of the user corresponds to any one of predetermined categories based on information on words that are likely used in expressions of negative automatic thoughts or on usages thereof, and that are pre-stored according to the predetermined categories into which automatic thoughts are classified, based on targets at which the negative automatic thoughts are prone to be directed,
wherein the message generation means generates, as the interactive message, a message representing a hint for allowing the user to input an adaptive thought or counterevidence according to the corresponding category prepared beforehand, when it is determined that the automatic thought, of the user, corresponds to any one of the predetermined categories as a result of the determination by the automatic thought category determination means.

11. The system for supporting correction of a cognitive distortion according to any one of claim 1 to claim 10, further comprising:
preconception determination means that determines whether a preconceived expression is present or not in an expression of an automatic thought of the user by extracting the preconceived expression included in information representing the automatic thought input by the user, based on information on words that are likely used in preconceived expressions or on usages thereof, pre-stored according to predetermined categories into which the preconceived expressions are classified according to expression contents thereof,
wherein the message generation means generates a message representing a hint according to the predetermined category into which the preconceived expression is classified, for allowing the user to input an adaptive thought or counterevidence when the preconception determination means determines that the preconceived expression is present.

12. The system for supporting correction of a cognitive distortion according to any one of claim 1 to claim 11, further executing a ground/counterevidence eliciting step comprising processing for outputting a message for prompting input of a ground of an automatic thought and processing for outputting a message for prompting input of counterevidence against the automatic thought,
wherein the message generation means generates, as the interactive message, a message representing a template for an expression of an adaptive thought as a hint for allowing the user to input an adaptive thought using a ground or the ground and counterevidence specified by information input by the user.

13. The system for supporting correction of a cognitive distortion according to any one of claim 1 to claim 12, further comprising:
vagueness determination means that determines whether a vague expression is present or not in an expression of an automatic thought of the user by extracting the vague expression included in information representing the automatic thought input by the user, based on information on pre-stored vague expressions,
wherein the message generation means generates, as the interactive message, a message for pointing out the vague expression when the vagueness determination means determines that the vague expression is present.

14. The system for supporting correction of a cognitive distortion according to claim 13, wherein
the message generation means further generates, as the interactive message, a message for proposing to correct vague expression to have an end in definite form with reference to a way of expression of an automatic thought when the vague expression determination means determines that the vague expression is present.

15. The system for supporting correction of a cognitive distortion according to any one of claim 1 to claim 14, further comprising:
contradiction determination means that determines the presence or absence of contradiction between the automatic thought and the feeling, based on an automatic thought category in which information representing the automatic thought of the user or the automatic thought of the user is classified into a predetermined category, and on a feeling category in which information representing the user's feeling or the user's feeling, when the automatic thought is generated, is classified into a predetermined category,
wherein the message generation means generates, as the interactive message, a message for pointing out contradiction when the contradiction determination means determines that there is the contradiction between the automatic thought and the feeling.

16. The system for supporting correction of a cognitive distortion according to claim 15, wherein
the message generation means generates, as the interactive message, a message for reconfirming the automatic thought or the feeling or for delving into the automatic thought or the feeling when the contradiction determination means determines that the contradiction is present between the automatic thought and the feeling.

17. The system for supporting correction of a cognitive distortion according to any one of claim 1 to claim 16, comprising:
the preconception determination means that determines whether a preconceived expression is present or not in an expression of an automatic thought of the user by extracting the preconceived expression included in information representing the automatic thought input by the user, based on information on pre-stored preconceived expressions,
wherein the message generation means generates, as the interactive message, a message for pointing out the preconceived expression when the preconception determination means determines that the preconceived expression is present.

18. The system for supporting correction of a cognitive distortion according to any one of claim 5 to claim 7, wherein the self/others determination means carries out at least any one determination of, self/others determination for determining whether a subject in a sentence comprising the extracted worry term or feeling term is a self or not, negative determination for determining whether the worry term or the feeling term is negated or not, interrogative determination for determining whether the sentence comprising the worry term or the feeling term is an interrogative sentence or not, conjectural determination for determining whether the worry term or the feeling language is conjectural or not, and conditional determination for determining whether the worry term or the feeling language is a conditional term or not, to determine whether the worry term or the feeling term represents an event actually occurred in the user or a feeling generated in the user.

19. The system for supporting correction of a cognitive distortion according to any one of claim 5 to claim 7, wherein
the self/others determination means uses a machine learning module that learned, in advance, a result of person's determination, for a sentence comprising a phrase or expression that can be the worry term or the feeling term, whether or not the phrase or the expression is a matter actually occurred in the user, to determine whether or not the extracted worry term or feeling term represents an event actually occurred in the user or a feeling generated in the user.

20. The system for supporting correction of a cognitive distortion according to claim 18, wherein
the self/others determination means determines, as the self/others determination, whether the subject in the sentence is a self or not, based on the genitive of a speaker and on the voice of a verb in the sentence comprising the extracted worry term or feeling term.

21. The system for supporting correction of a cognitive distortion according to claim 18, wherein
the self/others determination means determines, as the negative determination, whether the extracted worry term or feeling term is negated or not based on the number of negative expressions appearing behind the extracted worry term or feeling term.

22. The system for supporting correction of a cognitive distortion according to claim 18, wherein
the self/others determination means determines, as the interrogative determination, whether the worry term or the feeling term is included in an interrogative sentence or not based on whether or not an interrogative expression appears behind the extracted worry term or feeling term.

23. The system for supporting correction of a cognitive distortion according to claim 18, wherein
the self/others determination means determines, as the conjectural determination, whether the worry term or the feeling term is conjectural or not based on whether or not a conjectural expression appears behind the extracted worry term or feeling term.

24. The system for supporting correction of a cognitive distortion according to claim 18, wherein
the self/others determination means determines, as the conditional determination, whether the worry term or the feeling term is a conditional term or not based on whether or not a term which constitutes a conditional clause is present in a character string behind the extracted worry term or feeling term.

25. A system for supporting correction of a cognitive distortion, which is an interactive system that executes an automatic thought eliciting step comprising processing for outputting a message for prompting input of an automatic thought which is a thought generated in a user in a situation in which a user's feeling is perturbed and an adaptive thought eliciting step comprising processing for outputting a message for prompting input of an adaptive thought which is a thought that can be currently taken by the user with regard to the situation,
wherein the system comprises flow controlling means that controls a sequence of execution of each step,
wherein the flow controlling means carries out at least the automatic thought eliciting step and the adaptive thought eliciting step in this sequence as a basic flow, and carries out flow control of, discontinuing the automatic thought eliciting step or the adaptive thought eliciting step, when determining that user input does not proceed in the automatic thought eliciting step or the adaptive thought eliciting step, and proceeding to a predetermined step comprising processing for outputting a message for prompting input of information for an opportunity or hint for input by a user in the discontinued step.

26. The system for supporting correction of a cognitive distortion according to claim 25, further executing a mood eliciting step comprising processing for outputting a message for prompting input of a feeling generated in a user in a situation in which a user's feeling is perturbed,
wherein the flow controlling means carries out flow control of proceeding to the mood eliciting step when determining that user input does not proceed in the automatic thought eliciting step.

27. The system for supporting correction of a cognitive distortion according to claim 25 or claim 26, further executing a ground/counterevidence eliciting step comprising processing for outputting a message for prompting input of a ground of the automatic thought and processing for outputting a message for prompting input of counterevidence against the automatic thought,
wherein the flow controlling means carries out flow control of proceeding to the ground/counterevidence eliciting step when determining that user input does not proceed in the adaptive thought eliciting step.

28. A system for supporting correction of a cognitive distortion, comprising a screen for eliciting, from a user, information about a situation in which a user's feeling is perturbed,
wherein the screen comprises an entry field for writing a scene and an entry field for writing an event, which are separately disposed.

29. A system for supporting correction of a cognitive distortion, comprising a screen for eliciting, from a user, information about a situation that a user's feeling is perturbed,
wherein the screen comprises an entry field in which the user can make an entry and an apparent space available for the entry is limited to a size with not more than the predetermined number of characters.

30. A system for supporting correction of a cognitive distortion, comprising a screen for eliciting, from a user, an automatic thought which is a thought generated in the user in a situation in which a user's feeling is perturbed,
wherein the screen comprises a screen comprising a plurality of entry fields for allowing a user to enter an automatic thought and a screen comprising a selection function for, when a plurality of automatic thoughts are entered, selecting one of the automatic thoughts.

31. A system for supporting correction of a cognitive distortion, comprising a screen for eliciting, from a user, an adaptive thought which is a thought that can be currently taken by the user with regard to a situation in which a user's feeling is perturbed,
wherein an example sentence of the adaptive thought, which is generated from a ground which is a reason for generating an automatic thought which has been previously acquired from the user and is a thought generated in the user in the situation in which the user's feeling is perturbed, or the ground and counterevidence against the automatic thought, is displayed on the screen.

32. A system for supporting correction of a cognitive distortion, comprising a screen for eliciting, from a user, a ground of an automatic thought which is a thought generated in the user in a situation in which a user's feeling is perturbed,
wherein a sentence in which a copulative conjunction and a summarization expression of an automatic thought are joined is displayed in form of following an entry field for a ground on the screen.

33. A method for eliciting user consciousness information, applied to an interactive system for supporting correction of a cognitive distortion, that executes an automatic thought eliciting step comprising processing for outputting a message for prompting input of an automatic thought which is a thought generated in a user in a situation in which a user's feeling is perturbed, and an adaptive thought eliciting step comprising processing for outputting a message for prompting input of an adaptive thought which is a thought that can be currently taken by the user with regard to the situation,
the method comprising:
generating an interactive message which is a message for eliciting necessary information from the user by utilizing information previously input by the user in at least one step of the steps; and
outputting the generated interactive message.

34. The method for eliciting user consciousness information according to claim 33, further comprising:
generating, as the interactive message, a message comprising content representing sympathy for the situation or state of the user using at least one of a worry term which is a phrase representing worry, and a feeling term which is a phrase representing a feeling, of the user, specified based on information input by the user.

35. The method for eliciting user consciousness information according to claim 33 or claim 34, further comprising:
generating, as the interactive message, a message comprising content for pointing out a vague expression, a preconceived expression, or contradiction included in information input by the user.

36. The method for eliciting user consciousness information according to any one of claim 33 to claim 35, further comprising:
generating, as the interactive message, a message comprising content for a hint or opportunity for allowing the user to input information on a purpose, generated based on information input by the user.

37. A method for eliciting user consciousness information, applied to an interactive system for supporting correction of a cognitive distortion, that executes an automatic thought eliciting step comprising processing for outputting a message for prompting input of an automatic thought which is a thought generated in a user in a situation in which a user's feeling is perturbed, and an adaptive thought eliciting step comprising processing for outputting a message for prompting input of an adaptive thought which is a thought that can be currently taken by the user with regard to the situation,
wherein flow controlling means that controls a sequence of execution of each step carries out the automatic thought eliciting step and the adaptive thought eliciting step in this sequence as a basic flow, and carries out flow control of, discontinuing the automatic thought eliciting step or the adaptive thought eliciting step, when determining that user input does not proceed in the step, and proceeding to a predetermined step including processing for outputting a message for prompting input of information for an opportunity or hint for input by a user in the discontinued step.

38. The method for eliciting user consciousness information according to claim 37, applied to a system for supporting correction of a cognitive distortion, executing a feeling eliciting step comprising processing for outputting a message for prompting input of a feeling generated in the user in the situation in which the user's feeling is perturbed,
wherein the flow controlling means that controls a sequence of execution of each step carries out flow control of proceeding to the feeling eliciting step when determining that user input does not proceed in the automatic thought eliciting step.

39. The method for eliciting user consciousness information according to claim 37 or claim 38, applied to an interactive system for supporting correction of a cognitive distortion, executing a ground/counterevidence eliciting step comprising processing for outputting a message for prompting input of a ground of the automatic thought and processing for outputting a message for prompting input of counterevidence against the automatic thought,
wherein the flow controlling means that controls a sequence of execution of each step carries out flow control of shifting to the ground/counterevidence eliciting step when determining that user input does not proceed in the adaptive thought eliciting step.

40. A program for supporting correction of a cognitive distortion, applied to an interactive system for supporting correction of a cognitive distortion, that executes an automatic thought eliciting step comprising processing for outputting a message for prompting input of an automatic thought which is a thought generated in a user in a situation in which a user's feeling is perturbed, and an adaptive thought eliciting step comprising processing for outputting a message for prompting input of an adaptive thought which is a thought that can be currently taken by the user with regard to the situation,
wherein the program allows a computer to execute:
message output processing for outputting an interactive message which is a message for eliciting necessary information from the user in each of the steps; and
message generation processing for generating the interactive message in at least one step of the steps by utilizing information previously input by the user.

41. The program for supporting correction of a cognitive distortion according to claim 40, wherein
the program further allows the computer to generate:
as the interactive message, a message comprising content representing sympathy for the situation or state of the user using at least one of a worry term which is a phrase representing worry, and a feeling term which is a phrase representing a feeling, of the user, specified based on information input by the user, in the message generation processing.

42. The program for supporting correction of a cognitive distortion according to claim 40 or claim 41, wherein
the program further allows the computer to generate:
as the interactive message, a message comprising content for pointing out a vague expression, a preconceived expression, or contradiction included in information input by the user, in the message generation processing.

43. The program for supporting correction of a cognitive distortion according to any one of claim 40 to claim 42, wherein
the program further allows the computer to generate:
as the interactive message, a message comprising content for a hint or opportunity for allowing the user to input information on a purpose, generated based on information input by the user, in the message generation processing.

44. A program for supporting correction of a cognitive distortion, applied to an interactive system for supporting correction of a cognitive distortion, that executes an automatic thought eliciting step comprising processing for outputting a message for prompting input of an automatic thought which is a thought generated in a user in a situation in which a user's feeling is perturbed, and an adaptive thought eliciting step comprising processing for outputting a message for prompting input of an adaptive thought which is a thought that can be currently taken by the user with regard to the situation,
wherein the program allows a computer to execute the automatic thought eliciting step and the adaptive thought eliciting step in this sequence as a basic flow, and to execute a flow control processing of discontinuing the automatic thought eliciting step or the adaptive thought eliciting step, when determining that user input does not proceed in the automatic thought eliciting step or the adaptive thought eliciting step, and proceeding to a predetermined step including processing for outputting a message for prompting input of information for an opportunity or hint for input by a user in the discontinued step.

45. The program for supporting correction of a cognitive distortion according to claim 44, applied to a system for supporting correction of a cognitive distortion, executing a feeling eliciting step comprising processing for outputting a message for prompting input of a feeling generated in the user in the situation in which the user's feeling is perturbed,
wherein the program allows a computer to execute:
the flow control processing of proceeding to the feeling eliciting step when determining that user input does not proceed in the automatic thought eliciting step.

46. The program for supporting correction of a cognitive distortion according to claim 44 or claim 45, applied to an interactive system for supporting correction of a cognitive distortion, executing a ground/counterevidence eliciting step comprising processing for outputting a message for prompting input of a ground of an automatic thought and processing for outputting a message for prompting input of counterevidence against the automatic thought,
wherein the program allows a computer to execute:
the flow control processing of proceeding to the ground/counterevidence eliciting step when determining that user input does not proceed in the adaptive thought eliciting step.
